# EUROPEAN PATENT APPLICATION

(11) **EP 4 578 940 A2**
(43) Date of publication of application: **02.07.2025**
(21) Application number: 25168831.3
(22) Date of filing: 24.02.2023
(51) Int. Cl.: C12N 1/20

(54) **USES OF BIFIDOBACTERIUM LONGUM TRANSITIONAL MICROORGANISM**

(30) Priority: 25.02.2022 EP 22158720; 01.11.2022 EP 22204950
(62) Divisional of application: 23709929.6
(71) Applicant: Société des Produits Nestlé S.A., 1800 Vevey (CH)
(72) Inventor: DELANNOY-BRUNO, Omar, 1007 Lausanne (CH); SAKWINSKA, Olga, 1009 Pully (CH); DUBOUX, Stéphane, 1162 St-Prex (CH)

(57) **Abstract**

The present invention relates to the use of a *Bifidobacterium longum* transitional microorganism to metabolise one or more glycan substrates selected from the group recited in any of Tables 1 to 3.

## Description

### Field of the Invention

The present invention is related to uses and methods comprising the use of a glycan substrate, optionally in combination with a *Bifidobacterium longum* transitional microorganism, to promote or assist the transition from a milk-based diet to solid food in an infant and/or in a young child.

### Background of the Invention

Nutrition plays a critical role in the development across all areas (including cognitive, motor, sensory, dentition, musculo-skeletal, immunity, and social development) in infants and young children. Further, the gastrointestinal or "gut" microbiome during infancy can play a significant role in the health and development of the infant both during infancy and later on in life (see e.g., Tanaka and Nakayama. 2017. Allergol. Int. 66(4): 515-522). Various factors, including diet, can significantly influence the microbiome structure and thus influence the health and development of an infant both during infancy and later in life. During infancy, a mammal, including a human, will transition from a diet that is composed of all or primarily a mother's milk to one of solid foods. This is referred to as the "transitional period", "transitional feeding period", or "weaning". As this occurs, significant changes in the gut microbiome structure can take place due the change in diet and other stressors during that time (see e.g., Vatanen et al., 2019. Nature Microbiology. 4:470-479; Dizzell et al., 2021. PLOS ONE. https://doi.org/10.1371/journal.pone.0248924; Moore and Townsend. 2019. Open Biol. Sep; 9(9):190128; Magne et al. 2006. FEMS Microbiology Ecology, 58(3): 563-571; and Edwards C.A. Ann Nutr Metab 2017;70:246-250). The change in microbiome structure can in turn impact the physiologic, cognitive, anatomical, health or other state or characteristic of the mammal. Although several studies have and are currently investigating the gut microbiome during infancy and young childhood, the gut microbiome and its impact on the immediate and lifelong health and well-being of the infant is far from being well characterized. Paralleling the lack of characterization and understanding of the gut microbiome in infancy and young childhood is also a paucity of compositions and formulations capable of facilitating a healthy gut microbiome appropriate for infant or young child use. As such, there exists a need for improved characterization and understanding of the gut microbiome and compositions and methods to support and/or establish a healthy gut microbiome, particularly in infants and young children.

Transition between fully milk-based diet, either breast-feeding or formula feeding, and solid foods rich in proteins and fibres results in an increase of bacterial numbers in the gut leading to the evolution in a microbial composition associated with adult individuals. Weaning is considered as a stressful and complex process and the disruption of gut microbiota can lead to microbiota dysbiosis that is linked to pathogenesis of both intestinal disorders, such as diarrhoea, IBD, IBS and coeliac disease and extra-intestinal disorders, such as allergies, asthma, metabolic syndrome, cardiovascular disease, and obesity. It would be desirable to reduce the stress induced by weaning to develop and maintain a heathy gut microbiota.

As such, there exists a need for improved characterization and understanding of the gut microbiome during the weaning. Additionally, there is a need to provide nutritional compositions and methods to support the transition between milk-based diet and solid in infants and young children.

Any reference to prior art documents in this specification is not to be considered an admission that such prior art is widely known or forms part of the common general knowledge in the field.

### Summary of the invention

The inventors have found *Bifidobacterium longum* subsp microorganisms of a clade that is present in the gut microbiome of the transitional feeding period of mammals, particularly humans. *B. longum* microorganisms belonging to this clade are referred to herein as *Bifidobacterium longum* transitional (hereinafter *B. longum* transitional). *B. longum* transitional strains NCC 5000, NCC 5001, NCC 5002, NCC 5003 and NCC 5004 were deposited with the Collection nationale de cultures de micro-organismes (CNCM), Institute Pasteur by SOCIÉTÉ DES PRODUITS NESTLÉ S.A according to Budapest Treaty on 11^{th} of May 2021 receiving the deposit numbers CNCM I-5683, CNCM I-5684, CNCM I-5685, CNCM I-5686 and CNCM I-5687, respectively. It is shown in the US provisional patent application 63/216127 that the *B. longum* transitional microorganisms are greater in relative abundance during the transitional feeding period (e.g. weaning period) than either *B. longum* subsp. *infantis* (*B. infantis*) or *B. longum* subsp *longum.* Indeed, the relative abundance of *B. longum* subsp *infantis* decreases at the beginning of the transitional feeding period until the end of the transitional feeding period while *B. longum* subsp *longum* begins to increase in abundance. Vatanen *et al.* demonstrated that this distinct *Bifidobacterium longum* clade expanded with introduction of solid foods and harbored enzymes for utilizing both breast milk and solid food substrates (Vatanen et al.; 2022, Cell 185, 1-18; published online 1 November 2022; https://doi.org/10.1016/j.cell.2022.10.011).

The *B. longum* transitional microorganisms encode a distinct profile of Carbohydrate-Active Enzymes (CAZymes). CAZymes relate to enzyme classes and families thereof that catalyze the breakdown, biosynthesis and/or modification of glycoconjugates, oligo- and polysaccharides. These CAZymes may be beneficially targeted during the weaning period. In particular, the *B. longum* transitional microorganisms preferentially metabolize particular glycan substrates by virtue of the CAZymes they encode. Such glycan substrates may be advantageous for *B. longum* transitional microorganism growth and/or function and may be advantageous in improving the ability of the infant or young child to transition from milk to solid foods during the weaning period.

In one aspect the present invention provides the use of a *Bifidobacterium longum* transitional microorganism to metabolize one or more glycan substrates, preferably wherein the glycan substrate is selected from the groups recited in any of Tables 1 to 3.

Suitably, the *Bifidobacterium longum* transitional microorganism may be used to promote or assist the transition from a milk-based diet to solid food in an infant and/or in a young child.

In another aspect, the invention relates to the use of a combination of a *Bifidobacterium longum* transitional microorganism and one or more glycan substrates to promote or assist the transition from a milk-based diet to solid food in an infant and/or in a young child, wherein the one or more glycan substrates is selected from the groups recited in any of Tables 1 to 3.

In a further aspect, the invention relates to the use of a combination of a *Bifidobacterium longum* transitional microorganism and one or more glycan substrates to increase short-chain fatty acids (SCFA) production by the gut microbiome in an infant and/or in a young child, wherein the one or more glycan substrates is selected from the groups recited in any of Tables 1 to 3.

In another aspect, the invention relates to the use of a combination of a *Bifidobacterium longum* transitional microorganism and one or more glycan substrates to modulate the gut microbiome, wherein the one or more glycan substrates is selected from the groups recited in any of Tables 1 to 3.

In a further aspect, the invention relates to the use of a glycan substrate to promote the growth of a *Bifidobacterium longum* transitional microorganism in the gut microbiota of an infant and/or of a young child, wherein the glycan substrate is selected from one or more of the glycan substrates selected from the group recited in any of Tables 1 to 3.

In another aspect the invention provides a method of promoting the growth of a *Bifidobacterium longum* transitional microorganism in the gut microbiota of an infant and/or of a young child, the method comprising administering to the infant and/or to the young child a composition comprising one or more glycan substrates selected from the group recited in any of Tables 1 to 3.

In a further aspect, the present invention provides a method of promoting/assisting the transition from a milk-based diet to solid food in an infant and/or in a young child, the method comprising administering to the infant and/or to the young child a combination of a *Bifidobacterium longum* transitional microorganism and one or more glycan substrates, wherein the one or more glycan substrates is selected from the groups recited in any of Tables 1 to 3.

In another aspect, the invention provides a composition for promoting/assisting the transition from a milk-based diet to solid food in an infant and/or in a young child comprising a *Bifidobacterium longum* transitional microorganism and one or more glycan substrates selected from the group recited in any of Tables 1 to 3.

### Brief Description of the Drawings

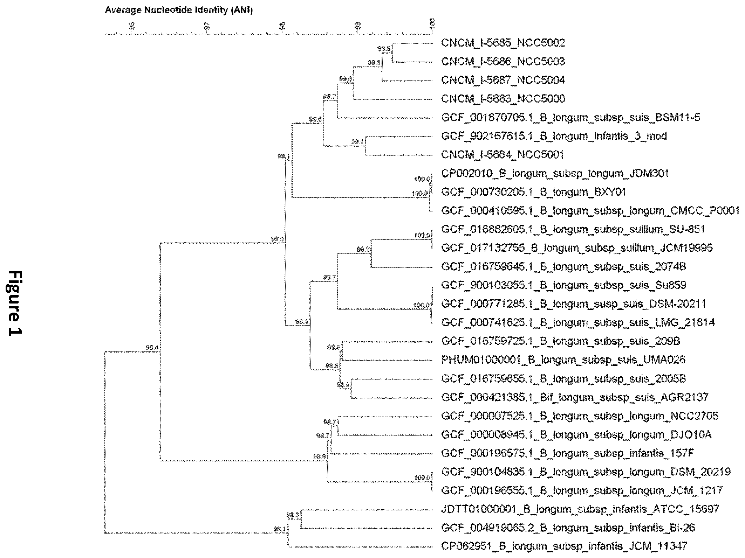

**Figure 1** shows the Average Nucleotide Identity (ANI) UPGMA based phylogenetic tree of strains belonging to the *B. longum* species. The scale represents the percentage of identity at each branch point.

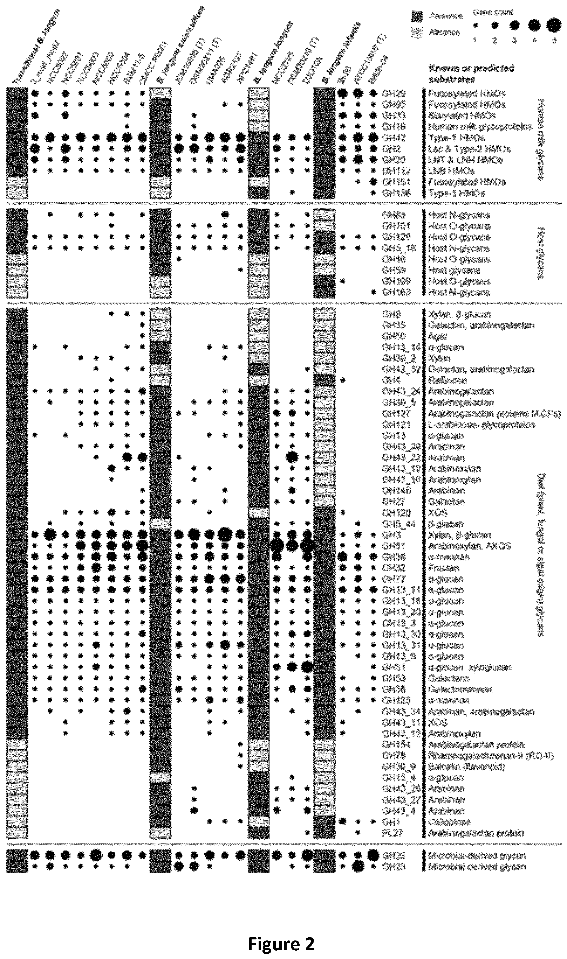

**Figure 2** shows a representation of glycoside hydrolases (GH) and polysaccharide lyases (PL) in the genomes of the *B. longum* clade. Heatmap shows presence (dark) and absence (light) of GH and PL genes, and the size of the circles represent the number of these genes per genome of a particular strain.

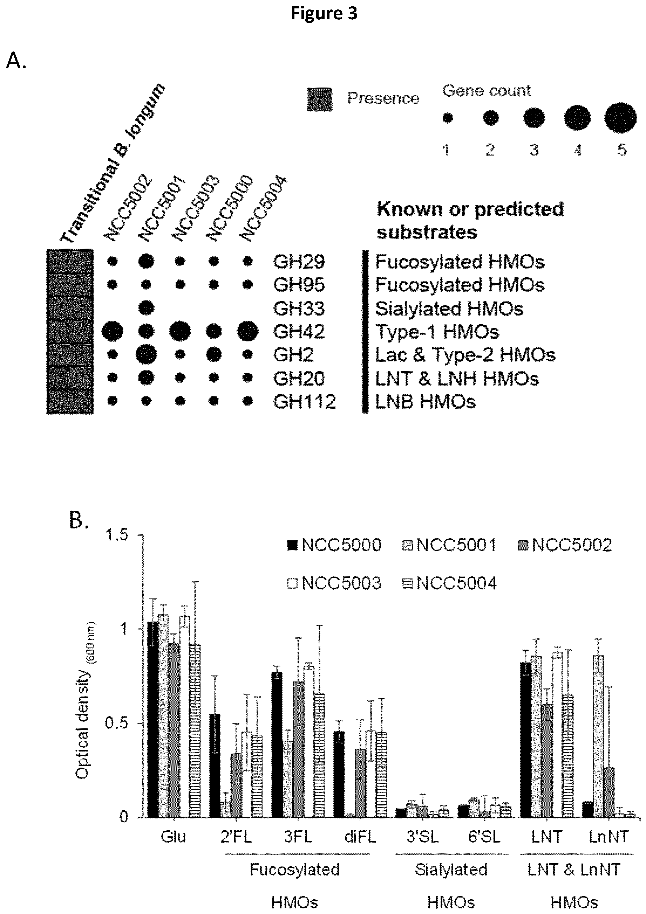

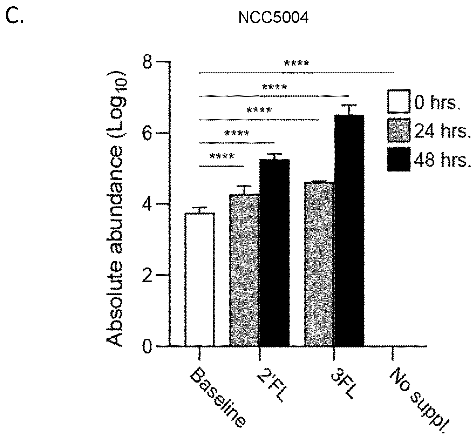

**Figure 3** shows that transitional *B. longum* isolates metabolize human milk oligosaccharides (HMOs) based on the CAZymes they encode. **(A)** Comparison of CAZyme profiles that target human milk glycans between the transitional *B. longum* group and five isolates within the group: NCC5002, NCC5001, NCC5003, NCC5000 and NCC5004. CAZymes are grouped based on their targeted glycan substrates including neutral fucosylated HMOs, sialylated HMOs, and neutral non-fucosylated HMOs. **(B)** Bar plots show optical density measures at 600 nm (± S.D.) of transitional *B. longum* isolates NCC5000, NCC5001, NCC5002, NCC5003, and NCC5004 individually grown on different HMOs. **(C)** Bar plots show qPCR absolute abundance (log₁₀) measurements (± S.D.) of transitional *B. longum* NCC5004 isolate supplemented to a fecal sample collected from a toddler human donor after 0 hr (baseline), 24 hr, and 48 hr in *in vitro* fermentation. Neutral fucosylated HMOs 2'FL and 3FL were individually added to the *in vitro* fermentation tubes as testing groups. Control groups were included for each of the HMOs tested were no transitional *B. longum* isolates were supplemented (No suppl. group at 48 hr timepoint). One-way ANOVA with Benjamini, Krieger and Yekutiel two-stage step-up method (false discovery rate-corrected) comparing each group to baseline: ****q < 0.0001.

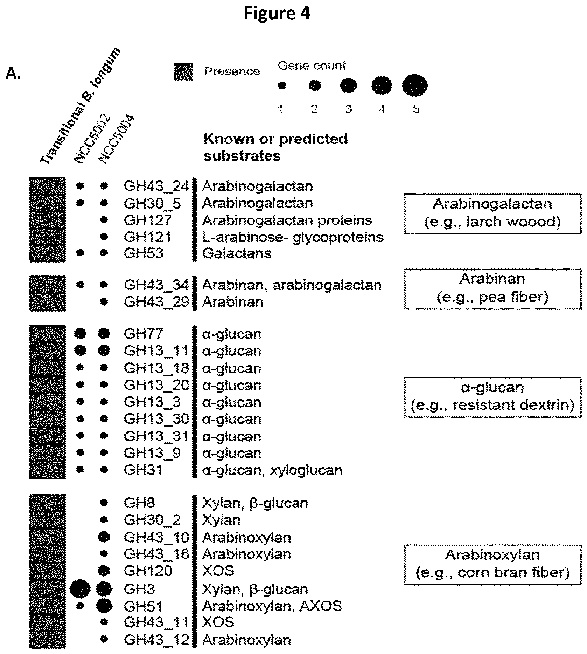

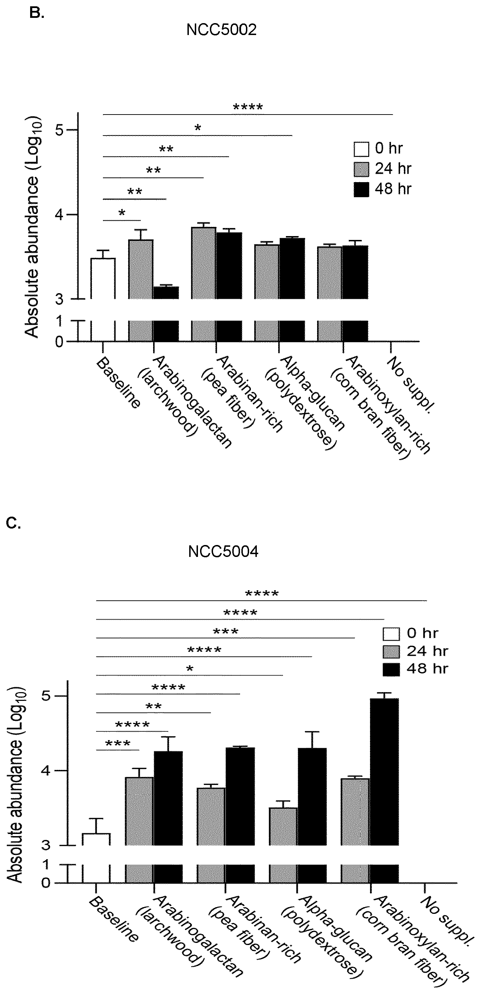

**Figure 4** shows that transitional *B. longum* metabolize specific glycan substrates based on the CAZymes they encode. **(A)** Comparison of CAZyme profiles that target plant fiber glycans between the transitional *B. longum* group and two isolates within the group: NCC5002 and NCC5004. CAZymes are grouped based on their targeted glycan substrates including arabinogalactan, arabinan, alpha-glucan and arabinoxylan. Bar plots show qPCR absolute abundance (log₁₀) measures (± S.E.M) of transitional *B. longum* isolates NCC5002 **(B)** and NCC5004 **(C)** supplemented to a fecal sample collected from a toddler human donor after 0 hr (baseline), 24 hr, and 48 hr in *in vitro* fermentation. Isolate-specific primers for a qPCR were used for quantification of isolates in the complex fecal community. Larch wood fiber (enriched in arabinogalactan), pea fiber (enriched in arabinan), polydextrose (enriched in the alpha-glucan resistant dextrin), and corn bran fiber (enriched in arabinoxylan) were individually added to the *in vitro* fermentation tubes as testing groups. Control groups were included for each of the glycans tested were no transitional *B. longum* isolates were supplemented (No suppl. group at 48 hr timepoint). One-way ANOVA with Benjamini, Krieger and Yekutiel two-stage step-up method (false discovery rate-corrected) comparing each group to baseline: *q < 0.05, **q < 0.01, ***q < 0.001, ****q < 0.0001.

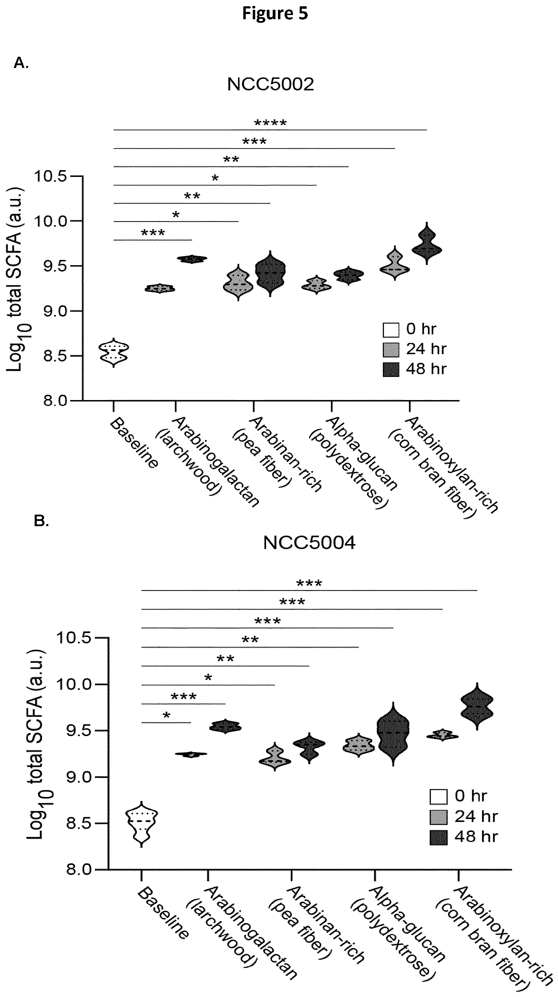

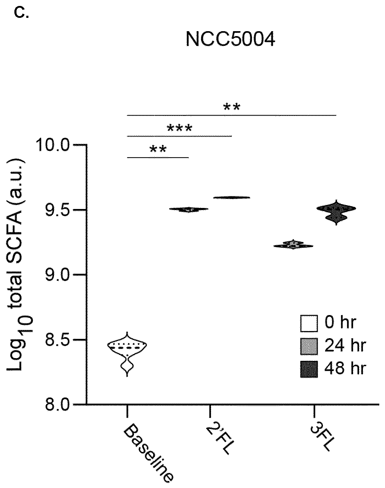

**Figure 5** **Combination of transitional *B. longum* with specific dietary glycans increase SCFA production in in vitro tube fermentation. (A)** Violin plots show log₁₀ total SCFA NMR measurements (sum of butyrate, acetate and propionate) in arbitrary units (a.u.) of supernatants from *in vitro* tube fermentations of a fecal sample collected from a toddler human donor after 0 hr (baseline), 24 hr, and 48 hr supplemented with dietary fiber glycans and transitional *B. longum* isolates NCC5002 **(A)** or NCC5004 **(B),** or supplemented with HMOs and transitional *B. longum* isolate NCC5004 (C). Kruskal-Wallis test with Benjamini, Krieger and Yekutiel two-stage step-up method (false discovery rate-corrected) comparing each group to baseline: *q < 0.05, **q < 0.01, ***q < 0.001, ****q < 0.0001.

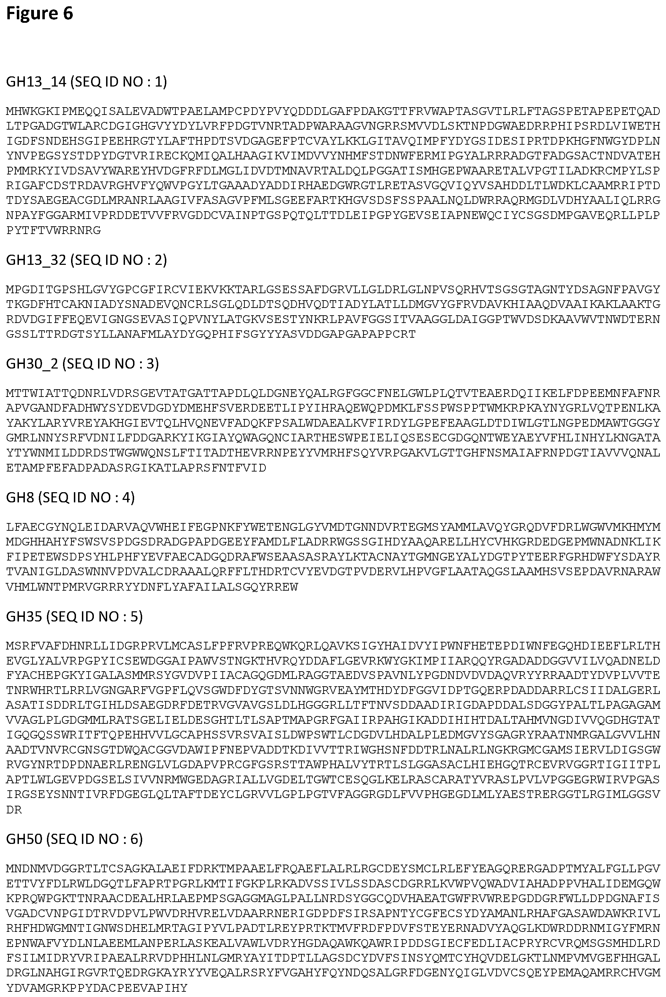

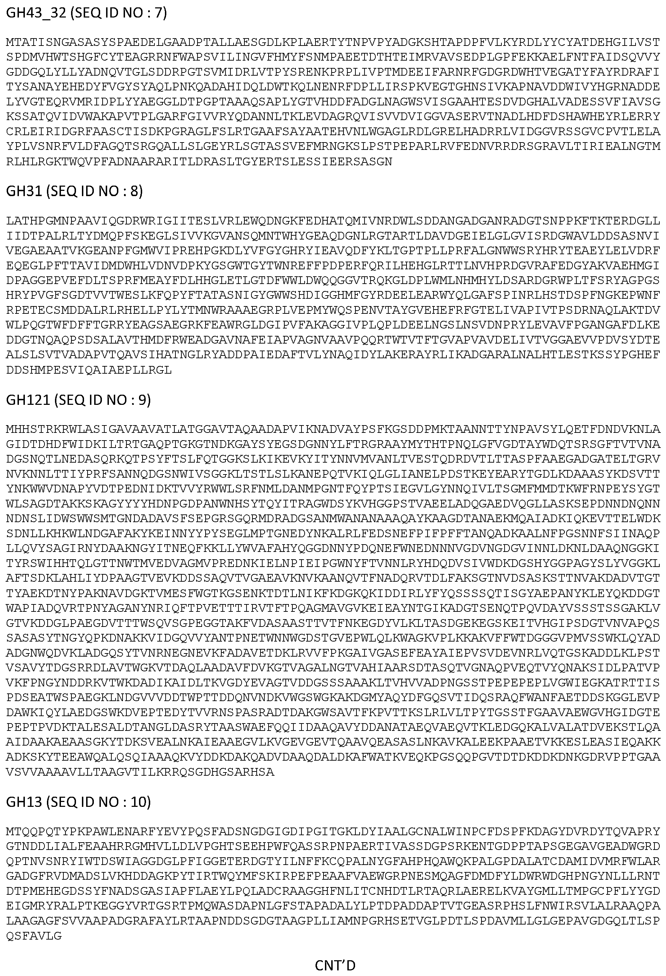

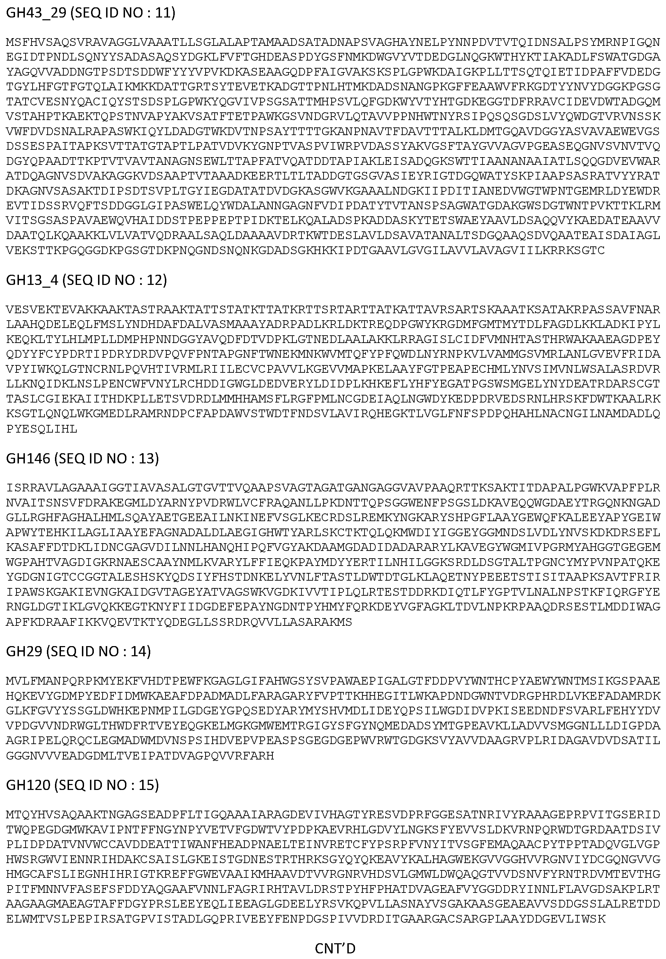

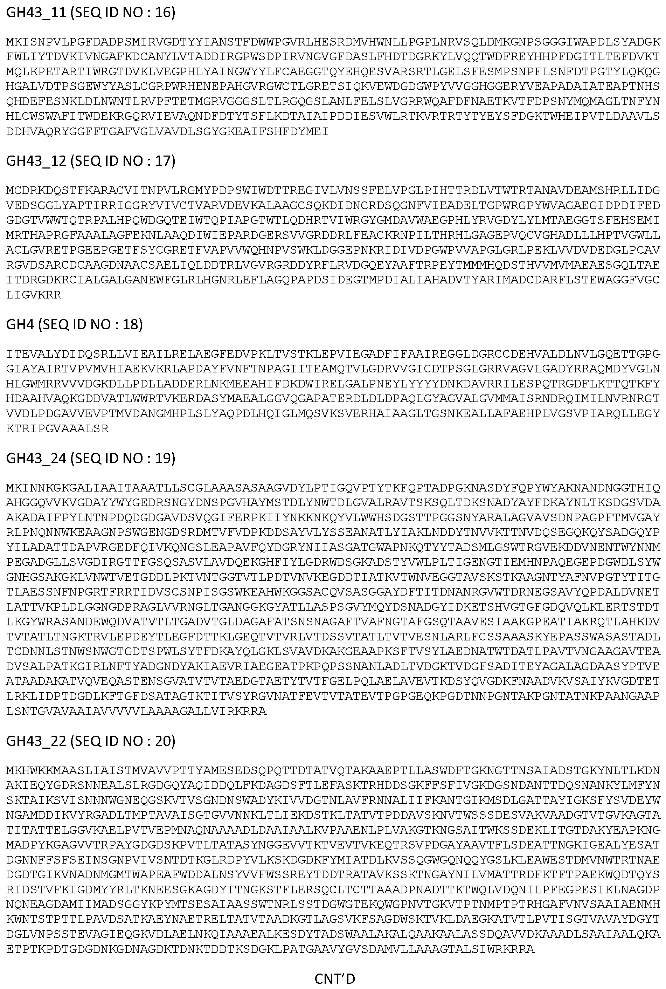

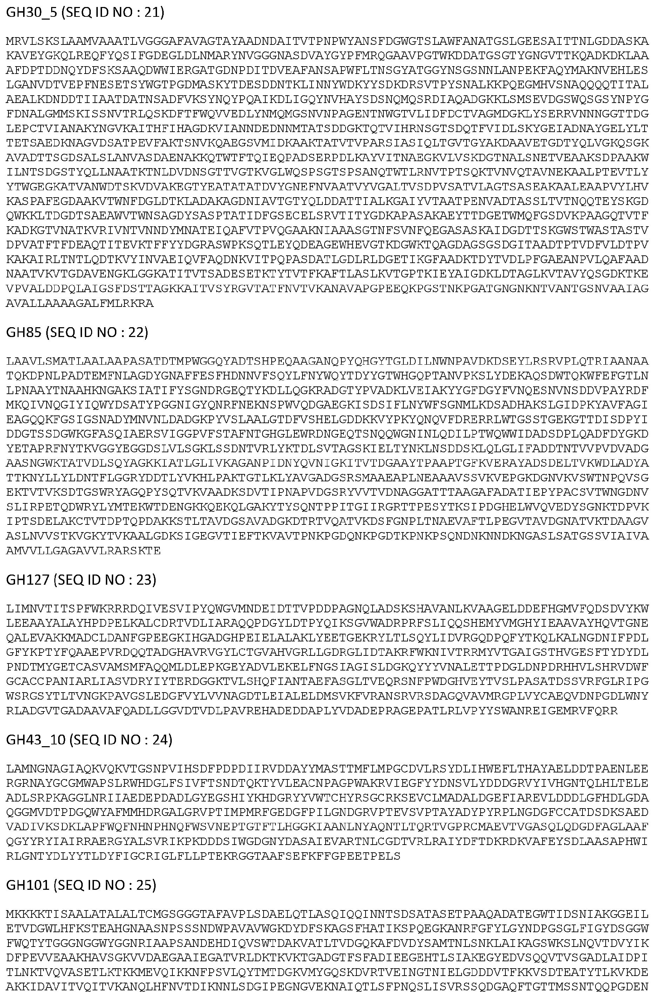

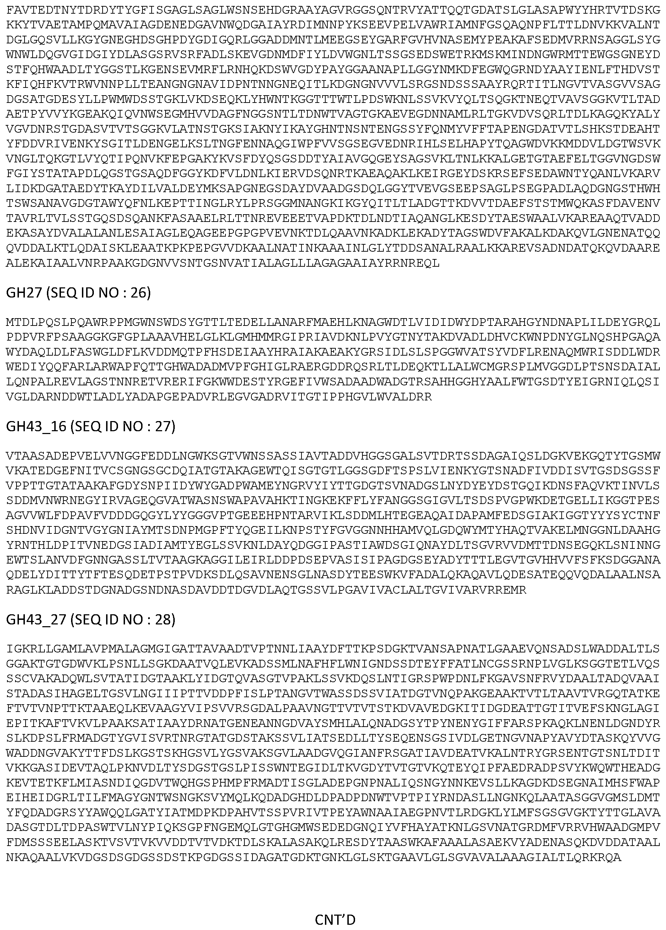

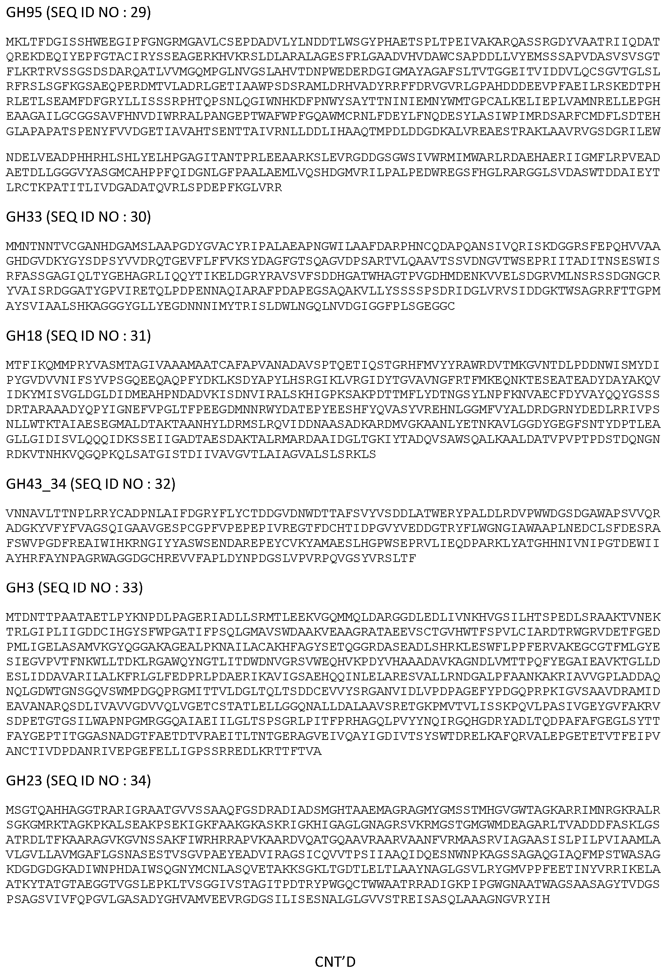

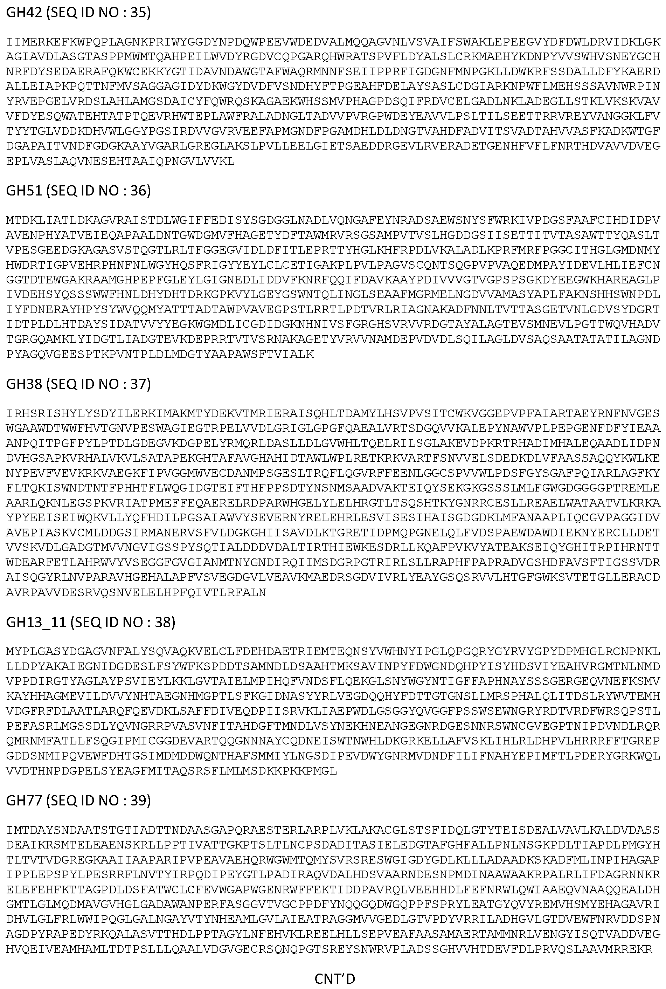

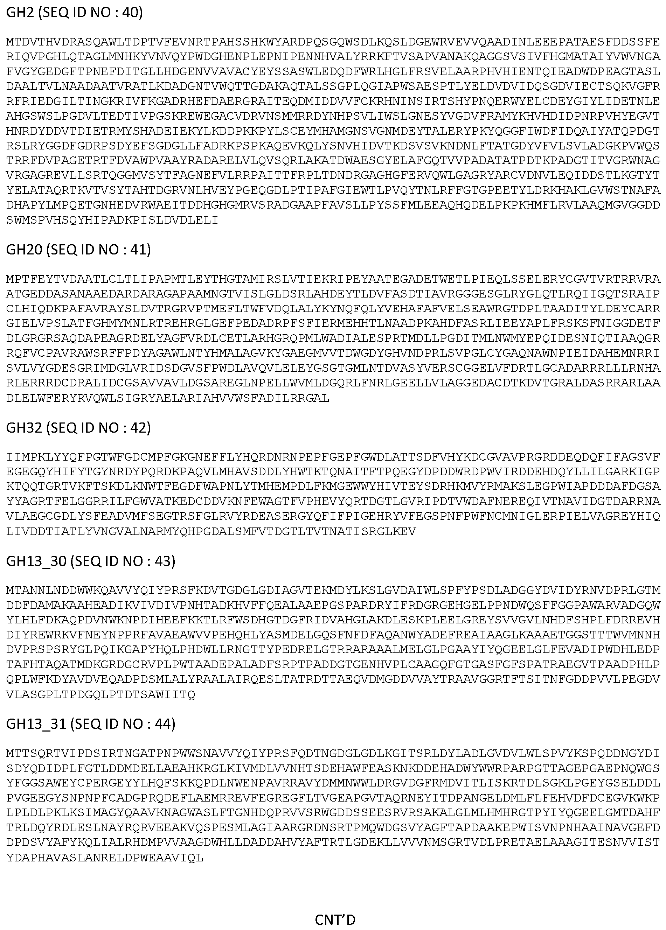

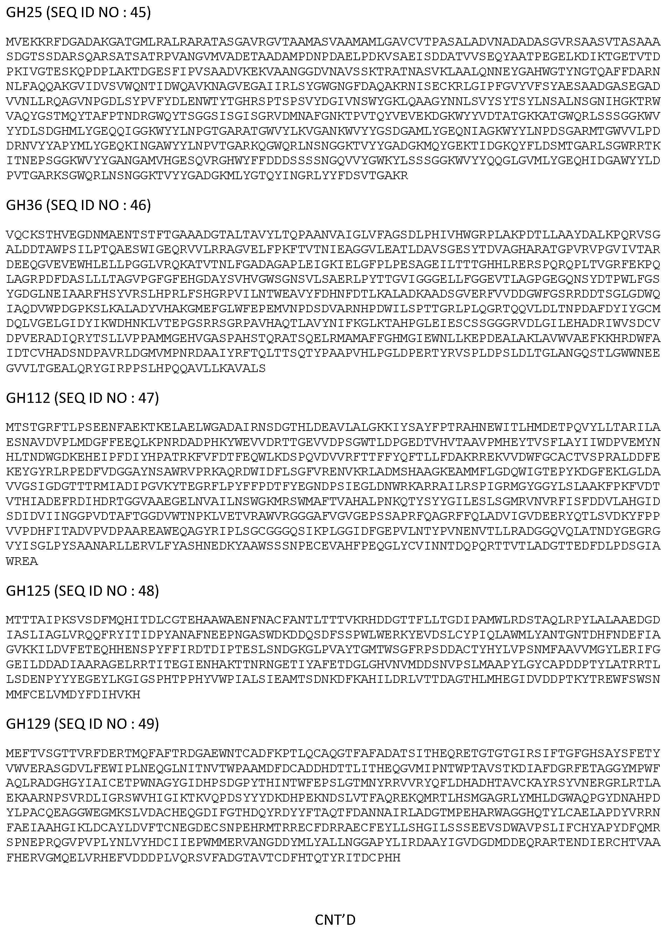

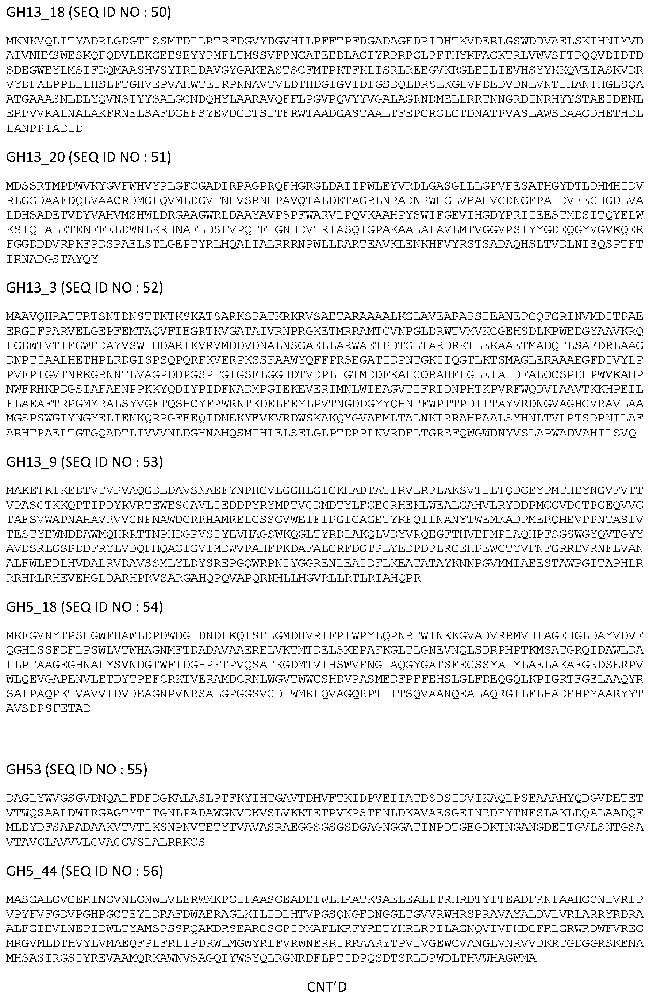

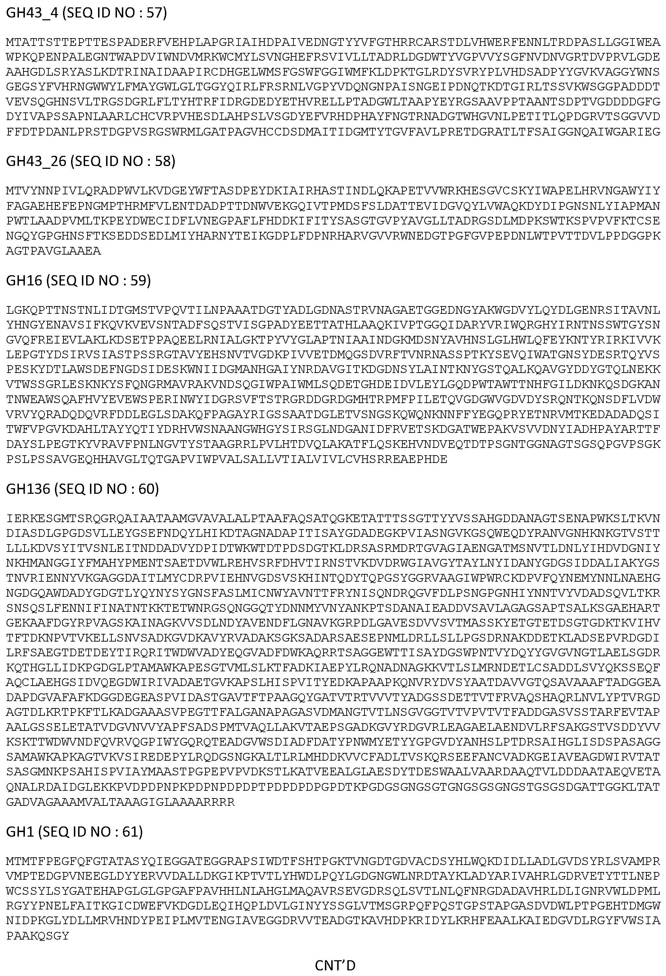

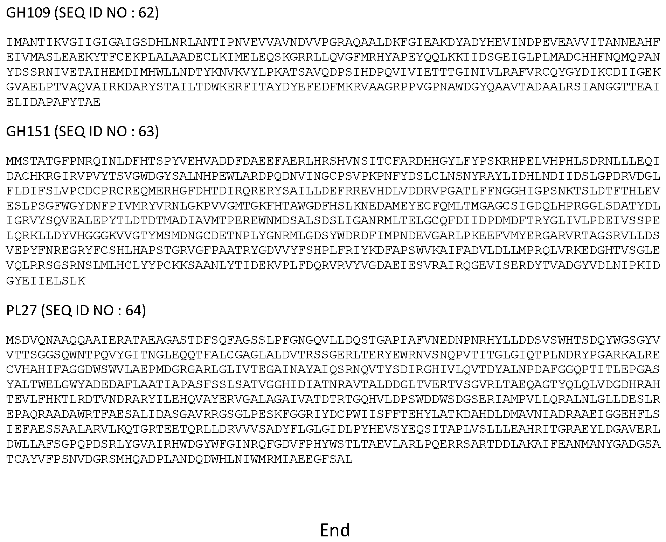

**Figure 6** shows representative CAZyme sequences.

### Detailed Description of the invention

All percentages are by weight unless otherwise stated.

The terms "about" or "approximatively" as used herein when referring to a measurable value such as a parameter, an amount, a temporal duration, and the like, are meant to encompass variations of and from the specific value, such as the variation of 1/-10% or less, 1/-5% or less, 1/-1% or less, and +/0.1% or less of and from the specific value, insofar such variations are appropriate to perform in the disclosed invention. It is to be understood that the value to which the modifier "about" or "approximately" refers is itself also specifically, and preferably, disclosed.

The terms "subject", "individual" and "patient" are used interchangeably to refer to a vertebrate, preferably a mammal, more preferably a human. Mammals include but are not limited to murines, simians, humans, farm animals, sport animals and pets.

The term "infant" means a human subject under the age of 12 months or an age equivalent non-human animal.

The terms "young child" or "toddler" as used herein may mean a human subject aged between 12 months and 5 years of age.

The expressions "complementary feeding period", "complementary period", "transitional period", "transitional feeding period" and "weaning period" can be interchangeably used and refer to the period during which the milk, either breast milk or formula, is substituted by other foods in the diet of an infant or a young child. The infant or the young child is typically moved or transitioned gradually from exclusive milk-feeding, either breast feeding or formula feeding, to mixed diet comprising milk and/or solid foods. The transitional period depends on the infant or young child but typically falls between about 4 months and about 18 months of age, such as between about 6 and about 18 months of age, but can in some instances extend up to about 24 months or more. For humans, the weaning period typically starts between 4 and 6 months of age and is considered completed once the infant and/or the young child is no longer fed with breast milk or infant formula, typically at about 24 months of age. In some embodiments, the weaning period is between 4 and 24 months.

The expressions "composition" or "nutritional composition" refer to any kind of composition or formulation that provides a nutritional benefit to an individual and that may be safely consumed by a human or an animal. Said nutritional composition may be in solid (e.g. powder), semisolid or liquid form and may comprise one or more macronutrients, micronutrients, food additives, water, etc. For instance, the nutritional composition may comprise the following macronutrients: a source of proteins, a source of lipids, a source of carbohydrates and any combination thereof. Furthermore, the nutritional composition may comprise the following micronutrients: vitamins, minerals, fiber, phytochemicals, antioxidants, prebiotics, probiotics, and any combination thereof. The composition may also contain food additives such as stabilizers (when provided in liquid or solid form) or emulsifiers (when provided in liquid form). The amount of the various ingredients (e.g. the oligosaccharides) can be expressed in g/100 g of composition on a dry weight basis when it is in a solid form, e.g. a powder, or as a concentration in g/L of the composition when it refers to a liquid form (this latter also encompasses liquid composition that may be obtained from a powder after reconstitution in a liquid such as milk, water, e.g. a reconstituted infant formula or follow-on/follow-up formula or infant cereal product or any other formulation designed for infant or young child nutrition). Generally, a nutritional composition can be formulated to be taken enterally, orally, parenterally, or intravenously, and it usually includes one of more nutrients selected from: a lipid or fat source, a protein source, and a carbohydrate source. Preferably, a nutritional composition is for oral use.

In a particular embodiment, the composition of the present invention is a "synthetic nutritional composition". The expression "synthetic nutritional composition" means a mixture obtained by chemical and/or biological means.

The expression "infant formula" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A "follow-up formula" or "follow-on formula" is given from the 6th month onwards. It constitutes the principal liquid element in the progressively diversified diet of this category of person.

The expression "baby food" means a foodstuff intended for particular nutritional use by infants or young children during the first years of life.

The expression "infant cereal composition" means a foodstuff intended for particular nutritional use by infants or young children during the first years of life.

The expression "growing-up milk" (or GUM) refers to a milk-based drink generally with added vitamins and minerals, that is intended for young children or children.

The terms "fortifier" refers to liquid or solid nutritional compositions suitable for fortifying or mixing with human milk, infant formula, growing-up milk or human breast milk fortified with other nutrients. Accordingly, the fortifier can be administered after dissolution in human breast milk, in infant formula, in growing-up milk or in human breast milk fortified with other nutrients or otherwise it can be administered as a stand-alone composition. When administered as a stand-alone composition, the milk fortifier can be also identified as being a "supplement".

The term "metabolize" is used herein to mean that a substrate can by broken down, adsorbed and/or utilized by a microorganism. For example, the substrate may promote and/or contribute to the growth and/or survival of the microorganism.

Suitably, the term "capable of metabolizing" may mean that the *B. longum* transitional strain encodes at least one CAZyme which is capable of utilizing the glycan substrate. For example, the CAZyme may be capable of catalyzing the hydrolysis of a glycosidic bond within the glycan substrate. Suitably, the *B. longum* transitional strain may encode at least one, at least two, at least three, at least four or at least five CAZymes that are capable of utilizing the glycan substrate. Suitably, the term "capable of metabolizing" may mean that the glycan substrate (or a fiber or ingredient comprising the glycan substrate) is capable of promoting growth and/or survival of the *B. longum* transitional strain (e.g. when added to an anaerobic culture of the *B. longum* transitional strain). Growth and/or survival of the *B. longum* transitional strain may be determined by measuring the abundance of 16S rDNA - for example using PCR methods. An illustrative assay for measuring growth of a *B. longum* transitional strain in the presence of glycan substrates (e.g. in the form of fiber) is provided in Example 2.

A "glycan substrate" refers to a glycan that can be metabolized by a microorganism. A glycan substrate may be, for example, a glycoconjugate, oligo- or polysaccharide. Glycoconjugate glycans may comprise N-linked glycans or O-linked glycans within glycoproteins and proteoglycans, or glycolipids. For example, an O-linked glycan may comprise a protein or peptide where the oxygen atom of a serine or threonine residue is linked to a monosaccharide, oligo- or polysaccharide as in the case with glycosaminoglycans (GAGs). Further examples of "glycan substrates" are cellulose, which is a glycan composed of β-1,4-linked D-glucose, and chitin, which is a glycan composed of β-1,4-linked N-acetyl-D-glucosamine. Glycans may be homo- or heteropolymers of monosaccharide residues and can be linear or branched. "Glycan substrate" as used herein encompasses, for example, oligosaccharides and polysaccharides.

The "oligosaccharide" may refer to a carbohydrate that has greater than 2 but relatively few monosaccharide units (typically 3, 4, 5, 6, and up to 10). Exemplary oligosaccharides include, but are not limited to, fructo-oligosaccharides, galacto-oligosaccharides (raffinose, stachyose, verbascose), maltooligosaccharides, gentio-oligosaccharides, cellooligosaccharides, milk oligosaccharides (e.g., those present in secretions from mammary glands), isomalto-oligosaccharides, lactosucrose, mannooligosaccharides, melibiose-derived oligosaccharides, pectic oligosaccharides, xylo-oligosaccharides.

The term "polysaccharide" may refer to a carbohydrate that has more than ten monosaccharide units. Exemplary polysaccharides include, but are not limited to, starch, arabinogalactan, laminarin, chrysolaminarin, xylan, arabinoxylan, mannan, fucoidan and galactomannan. It is to be understood that there is not a precise cut-off or distinction between the terms oligosaccharide and polysaccharide, nor is such a distinction necessary to practice the invention.

The term "glycosaminoglycan" (GAG) or mucopolysaccharide refers to long linear polysaccharides consisting of repeating disaccharide units (i.e. two-sugar units). The repeating two-sugar unit consists of a uronic sugar and an amino sugar, with the exception of keratan, where in the place of the uronic sugar it has galactose. GAGs are classified into four groups based on core disaccharide structures.

"Mucins", as used herein, may refer to a family of high molecular weight, heavily glycosylated proteins (glycoconjugates). Mucins' key characteristic is their ability to form gels; therefore they are a key component in most gel-like secretions, serving functions from lubrication to cell signalling to forming chemical barriers.

The term "HMO" or "HMOs" refers to human milk oligosaccharide(s). These carbohydrates are highly resistant to enzymatic hydrolysis, indicating they may display essential functions not directly related to their caloric value. It has been especially illustrated they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy the terminal position at the non-reducing ends. Depending on the presence of fucose and sialic acid in the oligosaccharide structure, the HMOs can be divided as non-fucosylated (neutral) or fucosylated (neutral) and sialylated (acidic) and non-sialylated molecules, respectively.

The term fibers is used herein to refer to carbohydrates that are indigestible by a human or animal. Such fibers are also discussed in relation to carbohydrates herein. Suitably, the fiber can be fermented by one or more *B. longum* transitional microorganisms provided in the present use or composition and/or within one or more regions in the gastrointestinal tract within an organism, such as a human or non-human animal. As used herein, the expressions "fiber" or "fibers" or "dietary fiber" or "dietary fibers" within the context of the present invention indicate the indigestible portion, in small intestine, of food derived from plants which comprises two main components: soluble fiber, which dissolves in water and insoluble fiber. Mixtures of fibers are comprised within the scope of the terms above mentioned. Soluble fiber is readily fermented in the colon into gases and physiologically active byproducts and can be prebiotic and viscous. Insoluble fiber does not dissolve in water, is metabolically inert and provides bulking, or it can be prebiotic and metabolically ferment in the large intestine. Chemically, dietary fiber consists of carbohydrate polymers with three or more monomeric units which are not hydrolyzed by endogenous enzymes in the small intestine such as arabinoxylans, cellulose, and many other plant components such as resistant starch, resistant dextrins, inulin, lignin, chitins, pectins, arabinans, arabinogalactans, galactans, xylans, beta-glucans, and oligosaccharides. Non-limiting examples of dietary fibers are: prebiotic fibers such as Fructo-oligosaccharides (FOS), inulin, galacto-oligosaccharides (GOS), fruit fiber, vegetable fiber, cereal fiber, resistant starch such as high amylose corn starch.

As used herein, "added fiber" or "added dietary fiber" indicates an ingredient mainly or totally constituted by fiber which is added to the complementary nutritional composition and whose content in fiber contributes to the total fiber content of the composition. The total fiber content of the complementary nutritional composition is provided by the sum of amount of fiber naturally present in ingredients used in the recipe (for example from whole grain cereal flour) plus amount of added fiber.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12).

The term "probiotic" means microbial cell preparation or components of microbial cells with a beneficial effect on the health or well-being of the host (Salminen S, Ouwehand A. Benno Y. et al. "Probiotics: how should they be defined" Trends Food Sci. Technol. 1999:10 107-10). The microbial cells are generally bacteria or yeasts.

The term "cfu" should be understood as colony forming unit.

The "gut microbiota" is the composition of microorganisms (including bacteria, archaea and fungi) that live in the digestive tract.

The term "gut microbiome" may encompass both the "gut microbiota" and their "theater of activity", which may include their structural elements (nucleic acid, proteins, lipids, polysaccharides), metabolites (signaling molecules, toxins, organic and inorganic molecules) and molecules produced by coexisting hosts and structured by the surrounding environmental conditions (Berg, G., et al., 2020. Microbiome, 8(1), pp.1-22).

The term "SCFA" means short chain fatty acid(s).

The expressions "to increase the short-chain fatty acids (SCFA) production" or "increasing the short chain fatty acids (SCFA) production" may mean that the amount of systemic and/or colonic SCFA is higher in an individual fed with the nutritional composition according to the present invention in comparison with a standard. The expressions may also mean that the amount of SCFA is increased in preclinical or *in vitro* models following treatment with the nutritional composition or glycan substrate compared to a control, untreated sample, or compared to a sample collected at baseline, prior to treatment with the nutritional composition or glycan substrate. The SCFA production may be measured by techniques known by the skilled person such as Gas-Liquid Chromatography. SCFA may be produced either by the *Bifidobacterium longum* transitional strains or by other members of the microbiota. Examples of SCFA are acetate, butyrate and propionate. Those metabolites have advantageous impacts or effects cross-feeding other members of the microbiota, for example acetate is the substrate used by butyrate-producer microorganisms. SCFA also has an advantageous direct effect on improving the permeability of gut barrier and on promoting immune development.

### Glycan substrate

The present invention is based, at least in part, on the inventor's determination that *Bifidobacterium longum* transitional microorganisms encode a profile of Carbohydrate-Active Enzymes (CAZymes), which may be beneficially targeted during the weaning period. Without wishing to be bound by theory, it is considered that targeting these CAZymes by, for example, providing suitable glycan substrates in the form of a prebiotic, may promote the growth and/or survival of the *Bifidobacterium longum* transitional microorganisms in the gut microbiota of an infant or young child and thus be beneficial during the weaning period.

In particular, the present inventors have determined the CAZymes encoded by each of the *Bifidobacterium longum* transitional strains NCC 5000, NCC 5001, NCC 5002, NCC 5003 and NCC 5004, which were deposited with the Institute Pasteur according to Budapest Treaty on 11^{th} of May 2021 receiving the deposit numbers CNCM I-5683, CNCM I-5684, CNCM I-5685, CNCM I-5686 and CNCM I-5687, respectively.

Carbohydrate-active enzymes (CAZymes) are responsible for the synthesis and breakdown of glycoconjugates, oligo- and polysaccharides. They typically correspond to 1-5% of the genes in the living organism. Glycoconjugates, oligo- and polysaccharides play essential roles in many biological functions, for example as structure and energy reserve components and in many intra- and intercellular events. The Carbohydrate Active Enzyme (CAZy) classification is a sequence-based family classification system that correlate with the structure and molecular mechanism of CAZymes (www.cazy.org).

CAZymes include glycoside hydrolyases (GH), glycosyltransferases (GT), polysaccharide lyases (PL), carbohydrate esterases (CE), and carbohydrate-binding module families (CBM)
Suitably, the CAZyme may be a glycoside hydrolyase (GH). GHs catalyze the hydrolysis of glycosidic bonds between two or more carbohydrates or between a carbohydrate and a non-carbohydrate moiety. In most cases, the hydrolysis of the glycosidic bond is catalyzed by two amino acid residues of the enzyme: a general acid (proton donor) and a nucleophile/base. Depending on the spatial position of these catalytic residues, hydrolysis occurs via overall retention or overall inversion of the anomeric configuration.

A GH classification system is provided by the CAZy classification. Herein, GHs are divided into families based on molecular function (e.g., GH1, GH2, GH3, GH4, etc.). These families are then further divided into subfamilies based on subgroups found within a family that share a more recent ancestor and, typically more uniform in molecular function (e.g., GH13_1, GH13_2, GH13_3, GH13_4, etc.).

Table 1 provides details of CAZymes that are unique to the *Bifidobacterium longum* transitional strains (i.e., not encoded by *Bifidobacterium longum suis*/*suillum, Bifidobacterium longum longum* or *Bifidobacterium longum infantis* strains). Table 1 also provides a summary of the glycan substrate metabolized by each CAZyme and illustrative dietary fiber sources/ingredients.

**Table 1**

| **CAZvme** | **Function** | **Glvcan substrate** | **Dietary fiber source** | **Potential fibre rich ingredient (targeted fibre)** | **Example of commercially available purified fibre** |
|---|---|---|---|---|---|
| | | | | | |
| | | | | | |
| | | | | | |
| GH8 | Chitosanase, cellulase, licheninase, endo-β-1,3(4)-glucanase, endo-1,4-β-xylanase, and reducing-end-xylose releasing exo-oligoxylanase. | β-1,4-glucosamine (chitosan), β-1,3(β-1,4)-glucan, β-1,4-xylan | Crustacean shells,fungi, cereals (whole grains) | Chitooligosaccharides and chitosan, wheat, rye, barley, oat, rice, corn, and sorghum (whole grains) | Chitosan, barley bran fiber, oat fiber, corn bran fiber |
| GH35 | β-galactosidase, exo-β-glucosaminidase, exo-β-1,4-galactanase, and β-1,3-galactosidase. | β-1,4(β-1,3)-galactose (arabinogalactan and galactan), β-1,3-galactose (agarans), α-1,3(β-1,4)-galactose (β-carrageenan) | Fruit and vegetable pectins, cereals (whole grains), or seaweed dietary fiber | Carrot, radish, tomato, barley, soybean, peach, pear, banana, seaweed | Carrot pectin, larch wood arabinogalactan, gum arabic, tomato fiber, carrageenan |
| GH50 | β-agarase. | β-1,3-galactose (agarans) | Seaweed dietary fiber | Red algae (*Palmaria palmata*) | Seaweed fiber |

Table 2 provides details of CAZymes that were present in at least one *Bifidobacterium longum* transitional strain but not present in at least one of the groups selected from the *Bifidobacterium longum suis*/*suillum, Bifidobacterium longum longum* or *Bifidobacterium longum infantis* strains presented in Figure 2. Table 2 also provides a summary of the glycan substrate metabolized by each CAZyme and illustrative dietary fiber sources/ingredients.

**Table 2**

| **CAZy me** | **Function** | **Glycan substrate** | **Dietary fiber source** | **Potential fibre rich ingredient (targeted fibre)** | **Example of commerciallyavailable purified fibre** |
|---|---|---|---|---|---|
| GH13_ 14 | Pullulanase, α-amylase, α-glucosidase | α-1,4(α-1,6)(α-1,3)-glucose (pullulan, dextrin, amylose, amylopectin) | Fungi, resistant starch from cereals, (whole grains), legumes, vegetables and roots | Filamentous fungus, corn, dextrin, polydextrose | Pullulan from Aureobasidium pullulans, polydextrose, high amylose starch, resistant dextrin |
| GH30_ 2 | β-xylosidase. | β-1,4-xylose (arabinoxylan, xylan) | Cereals (whole grains) | Wheat, rye, barley, oat, rice, corn, and sorghum (whole grains) | Wheat arabinoxylan, corn arabinoxylan, psyllium husk arabinoxylan, XOS |
| GH43_ 32 | β-D-galactofuranosidase | β-1,4(β-1,3)-galactose (arabinogalact an) | Fruit pectins and cereals (whole grains) | Carrot, radish, tomato, barley, soybean, peach, pear, banana | Carrot pectin, larch arabinogalactan, Gum arabic |
| | | | | | |
| GH121 | β-L-arabinobiosidase. | β-L-arabinopyrano se (branches in arabinogalacta n proteins). | Cereals (whole grains) and roots | Carrot, radish, tomato, barley, soybean, peach, pear, banana | Carrot pectin, larch arabinogalactan, Gum arabic |
| GH13 | α-amylase, pullulanase, cyclomaltodextrin glucanotransferase, cyclomaltodextrinas e, trehalose-6-phosphate hydrolase, oligo-α-glucosidase, maltogenic amylase, neopullulanase, α-glucosidase, maltotetraose-forming α-amylase, isoamylase, glucodextranase, maltohexaose-forming α-amylase, maltotriose-forming α-amylase, branching enzyme, trehalose synthase, 4-α-glucanotransferase, maltopentaose-forming α-amylase, amylosucrase, sucrose phosphorylase, malto-oligosyltrehalose trehalohydrolase, isomaltulose synthase, malto-oligosyltrehalose synthase, amylo-α-1,6-glucosidase, α-1,4-glucan: phosphate α-maltosyltransferase, 6'-P-sucrose phosphorylase, and amino acid transporter | Linear α-1,4-glucan (amylose and amylopectin) | Resistant-starch from cereals (whole grains), legumes, vegetables and roots | Corn, dextrin | High amylose starch, resistant dextrin, polydextrose |
| GH43_ 29 | β-xylosidase, α-L-arabinofuranosidase, and α-1,2-L-arabinofuranosidase | α-1,5(α-1,2)(α-1,3)-arabinofuranos e (arabinan, arabinoxylan), β-1,4-xylose (arabinoxylan) | Pectins from fruits, vegetables, legumes and roots; hemicellulose from cereals (whole grains) | Wheat, peas, sugarbeet, apple, citruses, rye, barley, oat, rice, corn, and sorghum (whole grains) | Wheat arabinoxylan, corn arabinoxylan, psyllium husk arabinoxylan, pea fiber, sugarbeet pectin, apple pectin, citrus pectin |
| | | | | | |
| GH146 | β-L-arabinofuranosidase | β-L-arabinofuranos e (branches in arabinogalacta n proteins and from pectin side-chains) | Pectins from fruits, vegetables, legumes and roots; hemicellulose from cereals (whole grains) | Carrot, radish, tomato, barley, soybean, peach, pear, banana | Carrot pectin, larch arabinogalactan, Gum arabic, pea fiber, sugarbeet pectin, apple pectin, citrus pectin |
| GH29 | α-L-fucosidase and α-1,3/1,4-L-fucosidase | Terminal α-L-fucosyl linkages | Human milk oligosaccharides | HMOs, brown algae PS (Fucoidan) | Fucoidan, fucosylated HMOs |
| GH120 | β-xylosidase | β-1,4-xylose (arabinoxylan); β-1,3(β-1,4)-xylose (xylan) | Cereals (whole grains) or seaweed dietary fiber | Wheat, rye, barley, oat, rice (non refined) | Wheat arabinoxylan, corn arabinoxylan, psyllium husk arabinoxylan |
| | | | | | |
| | | | | | |
| GH4 | Different functions: maltose-6-phosphate glucosidase, α-glucosidase, α-galactosidase, 6-phospho-β-glucosidase, α-glucuronidase, α-galacturonase, and palatinase. | Linear α-1,4-glucan (amylose and amylopectin) | Resistant-starch from cereals (whole grains), legumes, vegetables and roots | Corn | High amylose starch, resistant dextrin |
| GH43_ 24 | Exo-β-1,3-galactanase. | β-1,3-galactose (galactan, arabinogalacta n) | Fruit and vegetable pectins and cereals (whole grain) | Carrot, radish, tomato, barley, soybean, peach, pear, banana, apple | Apple pectin, carrot pectin, larch arabinogalactan, Gum arabic |
| GH43_ 22 | β-xylosidase. | β-1,4-xylose (arabinoxylan); β-1,3(β-1,4)-xylose (xylan) | Cereals (whole grains) or seaweed dietary fiber | Wheat, rye, barley, oat, rice, corn, and sorghum (non refined) | Wheat arabinoxylan, corn arabinoxylan, psyllium husk arabinoxylan |
| GH30_ 5 | Endo-β-1,6-galactanase. | β-1,6-galactose (galactan) | Fruit and vegetable pectins and cereals (whole grain) | Apple, banana, radish | Apple pectin, carrot pectin, larch arabinogalactan, Gum arabic |
| GH85 | Endo-β-N-acetylglucosaminid ase. | GlcNAc-β-1,4-GlcNac (N-linked glycans) | N-linked oligosaccharides from fruits, nuts, vegetables, cereals and animal sources. | N-glycosylated proteins (mainly animal source) | Hyaluronic acid, chondroitin sulfate, heparin/heparan |
| GH127 | β-L-arabinofuranosidase and 3-C-carboxy-5-deoxy-L-xylose (aceric acid) hydrolase. | β-L-arabinofuranos e (branches in arabinogalacta n proteins and from pectin side-chains) | Pectins from fruits, vegetables, legumes and roots; hemicellulose from cereals (whole grains) | Carrot, radish, tomato, barley, soybean, peach, pear, banana | Carrot pectin, larch arabinogalactan, Gum arabic |
| GH43_ 10 | β-xylosidase and α-L-arabinofuranosidase. | α-1,5(α-1,2)(α-1,3)-arabinofuranos e (arabinan, arabinoxylan), β-1,4-xylose (arabinoxylan) | Pectins from fruits, vegetables, legumes and roots; hemicellulose from cereals (whole grains) | Wheat, peas, sugarbeet, apple, citruses, rye, barley, oat, rice (whole grain) | Wheat arabinoxylan, corn arabinoxylan, psyllium husk arabinoxylan, pea fiber, sugarbeet pectin, apple pectin, citrus pectin |
| GH101 | Endo-α-N-acetylgalactosamini dase. | GalNAc; Galβ1-3GalNAc (host-glycans) | Mucin | | |
| GH27 | α-galactosidase, isomalto-dextranase, β-L-arabinopyranosidas e, and galactan:galactan galactosyltransferas e. | α-1,6-galactopyranos e (galactomanna ns); β-L-arabinopyrano se (branches in arabinogalacta n proteins and from pectin side-chains) | Gum (guar, locust bean, fenugreek alfalfa); galactooligosacch arides (GOS); pectins from fruits, vegetables, legumes and roots; hemicellulose from cereals (whole grains) | Soybean hulls, clusterbean (*Cyamopsis tetragonoloba L.Taub*) | Galactooligosac charides (GOS), larch arabinogalactan, Gum arabic |
| GH43_ 16 | β-xylosidase and α-L-arabinofuranosidase | α-1,5(α-1,2)(α-1,3)-arabinofuranos e (arabinan, arabinoxylan), β-1,4-xylose (arabinoxylan) | Pectins from fruits, vegetables, legumes and roots; hemicellulose from cereals (whole grains) | Wheat, peas, sugarbeet, apple, citruses, rye, barley, oat, rice (whole grain) | Wheat arabinoxylan, corn arabinoxylan, psyllium husk arabinoxylan, pea fiber, sugarbeet pectin, apple pectin, citrus pectin |
| | | | | | |
| GH95 | α-L-fucosidase, α-1,2-L-fucosidase, and α-L-galactosidase. | Terminal α-L-fucosyl linkages; α-1,6-galactopyranos e (galactomanna ns) | Human milk oligosaccharides | HMOs, brown algae PS (Fucoidan) (galactomannan in guar gum) | Fucoidan, fucosylated HMOs |
| GH33 | Sialidase or neuraminidase, trans-sialidase, anhydrosialidase, Kdo hydrolase, and 2-keto-3-deoxynononic acid hydrolase / KDNase. | Terminal α-2,3(α-2,6)-sialyl linkages | Human milk oligosaccharides | HMOs, animal source ingredients (glycoproteins and glycolipids) | Sialylated HMOs |
| GH18 | Chitinase, lysozyme, endo-β-N-acetylglucosaminid ase, peptidoglycan hydrolase with endo-β-N-acetylglucosaminid ase specificity, Nod factor hydrolase, xylanase inhibitor, concanavalin B, and narbonin. | β-1,4-N-acetyl-D-glucosamine (chitin); GlcNAc-β-1,4-GlcNac (N-linked glycans) | Crustacean shells and fungi; N-linked oligosaccharides from fruits, nuts, vegetables, and cereals | Chitin, chitosan in mushroom extracts (Namex) | Chitosan, chitooligosaccharides (COS) |
| GH5_4 4 | β-glycosidase. | β-glucose (β-glucans) | Cereals (whole grains) | Barley, oat | Oat fiber, barley fiber |
| | | | | | |

Table 3 provides details of CAZymes that were present in all *Bifidobacterium longum* strains analysed (i.e., *Bifidobacterium longum* transitional, *Bifidobacterium longum suis*/*suillum, Bifidobacterium longum longum* and *Bifidobacterium longum infantis*)*.* Table 3 also provides a summary of the glycan substrate metabolized by each CAZyme and illustrative dietary fiber sources/ingredients.

**Table 3**

| **CAZyme** | **Function** | **Glycan substrate** | **Dietary fiber source** |
|---|---|---|---|
| GH3 | Different functions: β-glucosidase, xylan 1,4-β-xylosidase, β-glucosylceramidase, β-N-acetylhexosaminidase, α-L-arabinofuranosidase, glucan 1,3-β-glucosidase, glucan 1,4-β-glucosidase, isoprimeverose-producing oligoxyloglucan hydrolase, coniferin β-glucosidase, exo-1,3-1,4-glucanase, and β-N-acetylglucosaminide phosphorylases. | β-1,3(β-1,4)-glucose (β-glucans) | Cereals (whole grains) |
| GH23 | Lysozyme type G, peptidoglycan lyase (also known in the literature as peptidoglycan lytic transglycosylase), and chitinase. | (GlcNAc-MurNAc)n (bacterial peptidoglycan) | No dietary source |
| GH42 | β-galactosidase and α-L-arabinopyranosidase. | α-arabinopyranose; β-1,4(β-1,3)-galactose (arabinogalactan and galactan) | Pectins from fruits, vegetables, legumes and roots; hemicellulose from cereals (whole grains), Type-1 HMOs |
| GH51 | Endoglucanase, endo-β-1,4-xylanase, β-xylosidase, and α-L-arabinofuranosidase. | β-1,3(β-1,4)-glucose (β-glucans), β-1,4-xylose (β-xylans) | Cereals (whole grains) |
| GH38 | α-mannosidase, mannosyl-oligosaccharide α-1,2-mannosidase, mannosyl-oligosaccharide α-1,3-1,6-mannosidase, α-2-O-mannosylglycerate hydrolase, and mannosyl-oligosaccharide α-1,3-mannosidase. | α-1,6-mannose (mannose oligosaccharides; MOS) | Manooligosaccharides (MOS), galactomannans |
| GH13_11 | Isoamylase. | α-1,6-glucan (amylopectin) | Resistant-starch from cereals (whole grains), legumes, vegetables and roots |
| GH77 | Amylomaltase or 4-α-glucanotransferase. | α-1,4-glucan (starch) | Resistant-starch from cereals (whole grains), legumes, vegetables and roots |
| GH2 | Different functions: β-galactosidase, β-mannosidase, β-glucuronidase, α-L-arabinofuranosidase, mannosylglycoprotein endo-β-mannosidase, exo-β-glucosaminidase, α-L-arabinopyranosidase, and β-galacturonidase. | β-1,3(β-1,4)(β-1,6)-galactose, β-1,3-galactobiose/triose (GOS, galactans and arabinogalactans) | Galactooligosaccharides (GOS); pectins from fruits, vegetables, legumes and roots; lactose and HMOs |
| GH20 | β-hexosaminidase, lacto-N-biosidase, β-1,6-N-acetylglucosaminidase, and β-6-SO3-N-acetylglucosaminidase. | Galβ1-3GlcNAcβ1-3Galβ1-4Glc (lacto-N-tetraose) | Neutral non-fucosylated HMOs |
| GH32 | Invertase, endo-inulinase, β-2,6-fructan 6-levanbiohydrolase, endo-levanase, exo-inulinase, fructan β-(2,1)-fractosidase/1-exohydrolase, fructan β-(2,6)-fractosidase/6-exohydrolase, sucrose:sucrose 1-fructosyltransferase, fructan:fructan 1-fructosyltransferase, sucrose:fructan 6-fructosyltransferase, fructan:fructan 6G-fructosyltransferase, levan fructosyltransferase, [retaining] sucrose:sucrose 6-fructosyltransferase (6-SST), and cycloinulo-oligosaccharide fructanotransferase | β-1,2(β-2,6)-D-fructofuranose (fructans like inulin, raffinose, levan, graminan). | Chicory root, agave, onion, jerusalem artichoke, oat |
| GH13_30 | α-glucosidase. | α-1,4-glucan (amylose and amylopectin) | Resistant-starch from cereals (whole grains), legumes, vegetables and roots |
| GH13_31 | Oligo-α-1,6-glucosidase and glucodextranase. | α-1,6-glucan (amylopectin) | Resistant-starch from cereals (whole grains), legumes, vegetables and roots |
| GH25 | Lysozyme | (GlcNAc-MurNAc)n (bacterial peptidoglycan) | No dietary source |
| GH36 | α-galactosidase, α-N-acetylgalactosaminidase, stachyose synthase, and raffinose synthase. | α-1,6-galactopyranose (galactomannans; raffinose and stachyose oligosaccharides) | Gum (guar, locust bean, fenugreek alfalfa); galactooligosaccharides (Alpha-GOS); vegetables and legumes |
| GH112 | Lacto-N-biose phosphorylase or galacto-N-biose phosphorylase, D-galactosyl-β-1,4-L-rhamnose phosphorylase, and galacto-N-biose/ lacto-N-biose phosphorylase. | Galβ1, 3GalNAc, GNB (galacto-N-biose); Galβ1, 3GlcNAc, LNB (lacto-N-biose I) | Neutral non-fucosylated HMOs |
| GH125 | Exo-α-1,6-mannosidase. | α-1,6-mannose (mannose oligosaccharides; MOS) | MOS |
| GH129 | α-N-acetylgalactosaminidase. | GalNAc; Galβ1-3GalNAc (host-glycans) | Mucins |
| GH13_18 | Sucrose phosphorylase and 6'-P-sucrose phosphorylase. | Sucrose | Sucrose and raffinose |
| GH13_20 | Cyclic a-1,6-maltosyl-maltose hydrolase; α-glycosidase hydrolyzing pullulan, starch and g-cyclodextrin; α-amylase; maltogenic α-amylase; neopullulanase; pullulanase; cyclomaltodextrinase | α-1,4(α-1,6)-glucan (amylose and amylopectin) | Resistant-starch from cereals (whole grains), legumes, vegetables and roots |
| GH13_3 | α-1,4-glucan and phosphate α-maltosyltransferase. | α-1,4-glucan (starch) | Resistant-starch from cereals (whole grains), legumes, vegetables and roots |
| GH13_9 | α-1,4-glucan branching enzyme. | α-1,4-glucan (amylose and amylopectin) | Resistant-starch from cereals (whole grains), legumes, vegetables and roots |
| GH5_18 | β-mannosidase. | Manβ1-4GlcNAc disaccharide (N-glycans) | Host glycans, glycoproteins. |
| GH31 | α-glucosidase, α-galactosidase, α-mannosidase, α-1,3-glucosidase, sucrase-isomaltase, α-xylosidase, α-glucan lyase, isomaltosyltransferase, oligosaccharide α-1,4-glucosyltransferase, and sulfoquinovosidase. | Linear α-1,4-glucan (amylose and amylopectin) | Resistant-starch from cereals (whole grains), legumes, vegetables and roots |
| GH53 | Endo-β-1,4-galactanase. | β-1,4-galactose (galactan, arabinogalactan) | Different sources of fruit and vegetable pectins and cereals (whole grain) |
| GH43_34 | α-L-arabinofuranosidase and β-D-galactofuranosidase | α-1,5(α-1,2)(α-1,3)-arabinofuranose (arabinan, arabinoxylan); β-1,4(β-1,3)-galactose (arabinogalactan and galactan) | Pectins from fruits, vegetables, legumes and roots; hemicellulose from cereals (whole grains) |
| GH43_11 | β-1,3-xylosidase, β-xylosidase, and α-L-arabinofuranosidase. | α-1,5(α-1,2)(α-1,3)-arabinofuranose (arabinan, arabinoxylan), β-1,4-xylose (arabinoxylan) | Pectins from fruits, vegetables, legumes and roots; hemicellulose from cereals (whole grains) |
| GH43_12 | β-xylosidase and α-L-arabinofuranosidase. | α-1,5(α-1,2)(α-1,3)-arabinofuranose (arabinan, arabinoxylan), β-1,4-xylose (arabinoxylan) | Pectins from fruits, vegetables, legumes and roots; hemicellulose from cereals (whole grains) |
| | | | |

Table 4 provides details of the CAZymes that are not encoded by *Bifidobacterium longum* transitional strains but are encoded by one or more of *Bifidobacterium longum suis*/*suillum, Bifidobacterium longum longum* and *Bifidobacterium longum infantis.*

**Table 4**

| **CAZyme** | **Function** | **Glycan substrate** |
|---|---|---|
| GH43_4 | [Inverting] endo-α-1,5-L-arabinanase. | α-1,5-arabinofuranose (arabinan) |
| GH43_26 | Exo-α-1,5-L-arabinofuranosidase and α-L-arabinofuranosidase. | α-1,5-arabinofuranose (arabinan) |
| GH16 | Xyloglucan:xyloglucosyltransferase, keratan-sulfate endo-1,4-β-galactosidase, endo-1,3-β-glucanase, endo-1,3(4)-β-glucanase, licheninase, β-agarase, k-carrageenase, xyloglucanase, endo-β-1,3-galactanase, β-porphyranase, hyaluronidase, endo-β-1,4-galactosidase, chitin β-1,6-glucanosyltransferase, and endo-β-1,4-galactosidase. | β-1,4-galactose; β-glucose (host-glycans) |
| GH136 | Lacto-N-biosidase. | lacto-N-tetraose, lacto-N-hexaose, lacto-N-fucopentaose I, and sialyllacto-N-tetraose |
| GH1 | Different functions: β-glucosidase, β-galactosidase, β-mannosidase, β-glucuronidase, β-xylosidase, β-D-fucosidase, phlorizin hydrolase, exo-β-1,4-glucanase, 6-phospho-β-galactosidase, 6-phospho-β-glucosidase, strictosidine β-glucosidase, lactase, amygdalin β-glucosidase, prunasin β-glucosidase, vicianin hydrolase, raucaffricine β-glucosidase, thioglucosidase, β-primeverosidase, isoflavonoid 7-O-β-apiosyl-β-glucosidase, ABA-specific β-glucosidase, DIMBOA β-glucosidase, β-glycosidase, and hydroxyisourate hydrolase. | α-1,5-arabinofuranose (arabino oligosaccharides; AOS), β-D-xylose (xylose oligosaccharides; XOS) |
| GH109 | α-N-acetylgalactosaminidase. | GalNAc; Galβ1-3GalNAc (host O-glycans) |
| GH151 | α-L-fucosidase. | α-L-fucosyl linkages |
| GH59 | β-galactosidase. | Host glycans |
| GH163 | Endo-β-N-acetylglucosaminidase. | GlcNAc-β-1,2-Man (host N-glycans) |
| GH154 | β-glucuronidase, β-1,6-D-glucuronidase, β-1,6-D-glucuronidase. | Arabinogalactan proteins (AGPs) containing β-1,6-D-glucuronic acid residues |
| GH78 | α-L-rhamnosidase, rhamnogalacturonan α-L-rhanmohydrolase, L-Rhap-α-1,3-D-Apif -specific α-1,3-L-rhamnosidase. | Rhamnogalacturonan |
| GH30_9 | Baicalin β-glucuronidase. | Baicalin |
| GH13_4 | Amylosucrase and sucrose hydrolase. | α-1,4-glucan (amylose and amylopectin) |
| GH43_27 | β-xylosidase and α-L-arabinofuranosidase. | α-1,5(α-1,2)(α-1,3)-arabinofuranose (arabinan, arabinoxylan), β-1,4-xylose (arabinoxylan) |
| PL27 | L-rhamnose-α-1,4-D-glucuronate lyase. | Terminal α-1-rhamnopyranosyl |

Representative sequences for the CAZymes listed in Tables 1-4 are shown in Figure 6. Suitably, the CAZyme referred to in any of Tables 1-4 may comprise or consist of the corresponding sequence shown in Figure 6. Suitably, the CAZyme may comprise or consist of a variant of the corresponding sequence shown in Figure 6, which variant retains at least one of the functions of the corresponding CAZyme as recited in Table 1-4. Suitably, the variant may provide each of the functional activities of the corresponding CAZyme as recited in Table 1-4. Suitably, the variant may comprise or consist of an amino acid sequence which has at least 70% sequence identity to the sequence listed in Figure 6, and retains at least one of the functional activities, preferably each of the functional activities, of the corresponding CAZyme as recited in Table 1-4. Suitably, the variant may comprise or consist of an amino acid sequence, which has at least 70%, at least 75%, at least 80%, at least 85%, at least 90%, at least 95%, at least 96%, at least 97%, at least 98%, or at least 99% sequence identity to the corresponding sequence listed in Figure 6. The variant retains at least one of the functional activities, preferably each of the functional activities, of the corresponding CAZyme as recited in Table 1-4.

Suitably, the present methods may comprise the use of a combination of glycan substrates selected from the groups recited in any of Tables 1 to 3.

The combination of glycan substrates may comprise at least 2, at least 4, at least 10, at least 20, at least 30, at least 40 or at least 50 of the glycan substrates selected from the groups recited in Tables 1 to 3. The combination may comprise each of the glycan substrates recited in Tables 1 to 3.

Suitably, the combination may comprise one or more glycan substrates selected from the group recited in Table 1 or Table 2.

The combination may comprise at least 2, at least 4, at least 10, at least 20, or at least 30 of the glycan substrates recited in Tables 1 and 2. The combination may comprise each of the glycan substrates recited in Tables 1 and 2.

Suitably, the glycan substrate can be provided directly by the diet, i.e., can be provided by one or more food item(s) consumed by an individual.

Suitably, the glycan substrate may comprise or consist of pectin, arabinogalactan and/or starch.

Suitably, the glycan substrate may comprise or consist of pectin.

Suitably, the glycan substrate may comprise or consist of arabinogalactan.

Suitably, the glycan substrate may comprise or consist of starch.

Suitably, the glycan substrate is provided in the form of a dietary fiber. For example, the dietary fiber may be a prebiotic fiber.

Suitably, the glycan substrate may be comprised in an ingredient, for example a dietary ingredient.

The ingredient containing one or several glycan substrates may be selected from the group consisting of purified polysaccharide or purified oligosaccharide, a dietary fiber ingredient, a semi-purified food ingredient, a raw food ingredient, a food additive, a HMO, a semi-purified or purified peptido-glycan.

The semi-purified food ingredient may be a fruit, vegetable or cereal extract.

The raw food ingredient may be a fruit, vegetable, cereal, algae or microalgae.

The food additive may be a guar gum or gum arabic.

The HMO may be a fucosylated oligosaccharide (i.e. an oligosaccharide having a fucose residue; e.g. 2' fucosyllactose (2-FL), 3-fucosyllactose (3-FL), difucosyllactose (DiFL), lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof), an N-acetylated oligosaccharide (e.g. LNT (lacto-N-tetraose), para-lacto-N-neohexaose (para-LNnH), LNnT (lacto-N-neotetraose), lacto-N-hexaose, lacto-N-neohexaose, para- lacto-N-hexaose, para-lacto-N-neohexaose, lacto-N-octaose, lacto-N- neooctaose, iso- lacto-N-octaose, para- lacto-N-octaose and lacto-N-decaose and any combinations thereof) and/or a sialylated oligosaccharide (e.g. 3'-sialyllactose (3-SL) or 6'-sialyllactose (6-SL)).

Suitably, the peptide-glycan may be a GAG.

Suitably, the glycan substrate may be comprised in a purified fiber.

Illustrative ingredients and/or purified fibers comprising suitable glycan substrates are provided in Tables 1 to 3. In particular, dietary fibers and/or ingredients that comprise a given glycan substrate are identified in the same row as the glycan substrate.

The pectin may be comprised in fruit or vegetable pectin. Accordingly, suitable ingredients comprising pectin include, but are not limited to, fruits (e.g., apple, pear), vegetables, legumes (peas), and roots (e.g., sugar beet). Suitable purified fibers comprising arabinogalactan include peach pectin. Suitably, the pectin extracted from sugar beet contains arabinan, galactans and arabinogalactans and may be provided as an ingredient.

The arabinogalactan may be comprised in fruit or vegetable pectin. Illustrative suitable ingredients comprising arabinogalactan include, but are not limited to, fruits, vegetables, whole grain cereals and seaweed dietary fiber. Suitable purified fibers comprising arabinogalactan include peach pectin, larch wood arabinogalactan, and Arabic gum. Suitably, the arabinogalactan may be provided in larch wood arabinogalactan.

The starch may be comprised in resistant-starch from cereals (whole grains), legumes, vegetables (e.g., corn) and roots (e.g., potato). Illustrative suitable ingredients comprising starch include, but are not limited to, corn. Suitable purified fibers comprising starch include high amylose starch and resistant dextrin. Suitably, the starch may be provided in a potato, corn or other ingredient. Suitably, the starch may be comprised in a potato ingredient.

In aspects of the invention in which a combination of a *Bifidobacterium longum* transitional microorganism and a glycan substrate are used, each may be selected such that the *Bifidobacterium longum* transitional microorganism is capable of metabolising the glycan substrate provided in the combination. Such a selection may be made, for example, by selecting a *Bifidobacterium longum* transitional microorganism that encodes a CAZyme from the same row of Tables 1-3 as the glycan substrate (or selecting an ingredient comprising said glycan substrate).

The combinations of the invention are not limited to requiring that the *Bifidobacterium longum* transitional microorganism is capable of metabolizing the glycan substrate provided in the combination. As such, any combinations of *Bifidobacterium longum* transitional microorganism(s) and glycan substrates disclosed herein are encompassed by the invention.

In one embodiment, the glycan substrate is not an HMO.

### Bifidobacterium longum transitional microorganism

Suitably, the *Bifidobacterium longum* transitional microorganism may encode one or more CAZymes selected from the groups recited in Table 1. Suitably, the *Bifidobacterium longum* transitional microorganism may encode one or two of CAZymes selected from the group recited in Table 1.

Suitably, the *Bifidobacterium longum* transitional microorganism encodes at least one CAZyme selected from the group recited in Table 1 and one or more of the CAZymes selected from the groups recited in Table 2 and 3. For example, the *Bifidobacterium longum* transitional microorganism may encode at least two, at least five, at least 10, at least 20 or at least 30 of the CAZymes selected from the groups recited in Table 2 and 3.

Suitably, *Bifidobacterium longum* transitional microorganism encodes (i) at least one CAZyme selected from the group recited in Table 1 and (ii) each of the CAZymes recited in Table 3 or each of the CAZymes recited in Table 3.

Suitably, *Bifidobacterium longum* transitional microorganism encodes (i) at least one CAZyme selected from the group recited in Table 1 and (ii) each of the CAZymes recited in Table 3 or each of the CAZymes recited in Table 3 apart from GH25.

Suitably, the *Bifidobacterium longum* transitional microorganism does not encode one or more of the CAZymes recited in Table 4. Suitably, the *Bifidobacterium longum* transitional microorganism does not encode any of the CAZymes recited in Table 4.

In some embodiments, a *Bifidobacterium longum* transitional microorganism has an Average Nucleotide Identity (ANI) of at least 98% with at least one *Bifidobacterium longum* strain selected in the group consisting of CNCM I-5683, CNCM I-5684, CNCM I-5685, CNCM I-5686 and CNCM I-5687, and any combination thereof. Suitably, the *Bifidobacterium longum* transitional microorganism encodes one or more CAZymes selected from the group recited in Table 1 and has an ANI of at least 98% with at least one *Bifidobacterium longum* strain selected in the group consisting of CNCM I-5683, CNCM I-5684, CNCM I-5685, CNCM I-5686 and CNCM I-5687, and any combination thereof. Suitably, the *Bifidobacterium longum* transitional microorganism encodes one or more GH31 CAZymes and has an ANI of at least 98% with at least one *Bifidobacterium longum* strain selected in the group consisting of CNCM I-5683, CNCM I-5684, CNCM I-5685, CNCM I-5686 and CNCM I-5687, and any combination thereof. In some embodiments, a *Bifidobacterium longum* transitional microorganism has an ANI of about 98% to 100% with at least one *Bifidobacterium longum* strain selected in the group consisting of CNCM I-5683, CNCM I-5684, CNCM I-5685, CNCM I-5686 and CNCM I-5687, and any combination thereof. In some embodiments, a *Bifidobacterium longum* transitional microorganism has an ANI of at least 98%, 98.1%, 98.2%, 98.3%, 98.4%, 98.5%, 98.6%, 98.6 %, 98.7 %, 98.8 %, 98.9 %, 99 %, 99.1 %, 99.2 %, 99.3 %, 99.4 %, 99.5 %, 99.6 %, 99.7 %, 99.8 %, 99.9 %, or 100 % with at least one *Bifidobacterium longum* strain selected in the group consisting of CNCM I-5683, CNCM I-5684, CNCM I-5685, CNCM I-5686 and CNCM I-5687, and any combination thereof. In some embodiments, a *Bifidobacterium longum* transitional microorganism has an ANI of at least 98.6%, of at least 98.6 %, of at least 98.7 %, of at least 98.8 %, of at least 98.9 %, of at least 99 %, of at least 99.1 %, of at least 99.2 %, of at least 99.3 %, of at least 99.4 %, of at least 99.5 %, of at least 99.6 %, of at least 99.7 %, of at least 99.8 %, of at least 99.9 % or of at least 100% with at least one *Bifidobacterium longum* strain selected in the group consisting of CNCM I-5683, CNCM I-5684, CNCM I-5685, CNCM I-5686 and CNCM I-5687, and any combination thereof.

Methods for sequencing microbial genomes are well known in the art (see e.g. Segerman; Front. Cell. Infect. Microbiol.; 2020; 10; Article 527102 & Donkor; Genes; 2013; 4(4); 556-572). By way of example, metagenomics methods may be used. Suitable metagenomics methods may be performed using shotgun sequencing data, for example. Suitable metogenomics methods are known in the art and include MetaPhlAn 3.0, for example (see Beghini et al.; eLife 2021;10: e65088; https://huttenhower.sph.harvard.edu/metaphlan).

The "Average Nucleotide Identity (ANI)" is a term of art that refers to a distance-based approach to delineate species based on pair-wise comparisons of their genome sequences and is an *in silico* alternative to the traditional DNA-DNA hybridization (DDH) techniques that have been used for phylogenetic definition of a species (Goris et al., 2007, "DNA-DNA hybridization values and their relationship to whole-genome sequence similarities", Int. J. Syst. Evol. Microbiol. 57: 81-91). Based on DDH, strains with greater than 70% relatedness would be considered to belong to the same species (see e.g., Wayne et al., 1987, Report of the Ad-Hoc-Committee on Reconciliation of Approaches to Bacterial Systematics. Int J Syst Bacteriol 37: 463-464). ANI is similar to the aforementioned 70% DDH cutoff value and can be used for species delineation. ANI has been evaluated in multiple labs and has become the gold standard for species delineation (see e.g., Kim et al., 2014, "Towards a taxonomic coherence between average nucleotide identity and 16S rRNA gene sequence similarity for species demarcation of prokaryotes", Int. J. Syst. Evol. Micr. 64: 346-351; Richter et al., 2009, "Shifting the genomic gold standard for the prokaryotic species definition", P Natl Acad Sci USA 106: 19126-19131; and Chan et al., 2012, "Defining bacterial species in the genomic era: insights from the genus Acinetobacter", Bmc. Microbiol. 12)).

The ANI of the shared genes between two strains is known to be a robust means to compare genetic relatedness among strains, and that ANI values of about 95% correspond to the 70% DNA-DNA hybridization standard for defining a species. See, e.g., Konstantinidis and Tiedje, Proc Natl Acad Sci USA, 102(7):2567-72 (2005); and Goris et al., Int Syst Evol Microbiol. 57(Pt 1):81-91 (2007). The ANI between two bacterial genomes is calculated from pair-wise comparisons of all sequences shared between any two strains and can be determined, for example, using any of a number of publicly available ANI tools, including but not limited to OrthoANI with usearch (Yoon et al. Antonie van Leeuwenhoek 110:1281-1286 (2017)); ANI Calculator, JSpecies (Richter and Rossello-Mora, Proc Natl Acad Sci USA 106:19126-19131 (2009)); and JSpeciesWS (Richter et al., Bioinformatics 32:929-931 (2016)). Other methods for determining the ANI of two genomes are known in the art. See, e.g., Konstantinidis, K. T. and Tiedje, J. M., Proc. Natl. Acad. Sci. U.S.A., 102: 2567-2572 (2005); and Varghese et al., Nucleic Acids Research, 43(14):6761-6771 (2015). In a particular embodiment, the ANI between two bacterial genomes can be determined, for example, by averaging the nucleotide identity of orthologous genes identified as bidirectional best hits (BBHs). Protein-coding genes of a first genome (Genome A) and second genome (Genome B) are compared at the nucleotide level using a similarity search tool, for example, NSimScan (Novichkov et al., Bioinformatics 32(15): 2380-23811 (2016)). The results are then filtered to retain only the BBHs that display at least 70% sequence identity over at least 70% of the length of the shorter sequence in each BBH pair. The ANI of Genome A to Genome B is defined as the sum of the percent identity times the alignment length for all BBHs, divided by the sum of the lengths of the BBH genes. These and ANI determination techniques are known in the art.

Suitably, a *Bifidobacterium longum* microorganism selected from the group consisting of CNCM I-5683, CNCM I-5684, CNCM I-5685, CNCM I-5686 and CNCM I-5687, represents the reference genome to which a microbial genome is compared.

Genome sequences for *B. longum* transitional strains NCC 5000 (CNCM I-5683), NCC 5001 (CNCM I-5684), NCC 5002 (CNCM I-5685), NCC 5003 (CNCM I-5686) and NCC 5004 (CNCM I-5687) are available via Joint Genome Project (JGI) Study number: Gs0156595 (hftps://genome.jgi.doe.gov/portal/). Analysis project numbers and taxon numbers for each genome are as follows:

| Strain | JGI analysis project | JGI taxon |
|---|---|---|
| B. longum NCC 5000 | Ga0527908 | 2951181949 |
| B. longum NCC 5001 | Ga0529016 | 2951184202 |
| B. longum NCC 5002 | Ga0529017 | 2951186501 |
| B. longum NCC 5003 | Ga0529018 | 2951188792 |
| B. longum NCC 5004 | Ga0529019 | 2951191018 |

In some embodiments, the *Bifidobacterium longum* transitional microorganism of the present invention is isolated from a human.

In some other embodiments, the *Bifidobacterium longum* transitional microorganism is not of the subspecies *Bifidobacterium longum subsp. longum* or *Bifidobacterium longum subsp. infantis.*

### Composition

The glycan substrate, or fiber or ingredient comprising the glycan substrate, may be provided in a composition.

The composition may be suitable for or may suitably be administered to an individual, for example an infant or a young child, in any suitable form such as a nutritional composition in a dosage unit (for example a tablet, a capsule, a sachet of powder, etc). The composition may be in powder, semi-liquid or liquid form. The composition may be added to a nutritional composition, an infant formula, a food composition, a supplement for infant or young child, a baby food, a follow-up formula, a growing-up milk, an infant cereal or a fortifier. In some embodiments, the composition of the present invention is an infant formula, a baby food, an infant cereal, a growing-up milk, a supplement or fortifier that may be intended for infants or young child.

The composition may further comprise a *Bifidobacterium longum* transitional microorganism as defined herein. The *Bifidobacterium longum* transitional microorganism can be included in the composition in an amount from about 10³ to 10¹² cfu of probiotic strain, more preferably between 10⁷ and 10¹² cfu such as between 10⁸ and 10¹⁰ cfu of probiotic strain per g of composition on a dry weight basis. In one embodiment, the *Bifidobacterium longum* transitional microorganism is viable. In another embodiment the *Bifidobacterium longum* transitional microorganism is non-replicating or inactivated. There may be both viable and inactivated *Bifidobacterium longum* transitional microorganisms in some other embodiments.

### Embodiments

The present invention provides the embodiments according to the following numbered clauses:
1. Use of a *Bifidobacterium longum* transitional microorganism to metabolize one or more glycan substrates, preferably wherein the glycan substrate is selected from the groups recited in any of Tables 1 to 3.
2. The use of a *Bifidobacterium longum* transitional microorganism according to clause 1, wherein the *Bifidobacterium longum* transitional microorganism is used to promote or assist the transition from a milk-based diet to solid food in an infant and/or in a young child.
3. The use according to clause 1 or 2, wherein the *Bifidobacterium longum* transitional microorganism is used to metabolize one or more glycan substrates recited in Table 1 or 2.
4. The use according to any preceding clause, wherein the *Bifidobacterium longum* transitional microorganism is used to metabolize one or more glycan substrates selected from pectin, arabinogalactan and/or starch.
5. The use according to any preceding clause, wherein the *Bifidobacterium longum* transitional microorganism encodes one or more CAZymes selected from the group recited in Table 1, preferably wherein the *Bifidobacterium longum* transitional microorganism further encodes one or more CAZymes selected from the groups recited in Table 2 and 3.
6. The use according to clause 5, wherein the *Bifidobacterium longum* transitional microorganism encodes (i) one or more CAZymes selected from the group recited in Table 1, and (ii) each of the CAZymes recited in Table 3 or each of the CAZymes recited in Table 3.
7. The use according to clause 6, wherein the *Bifidobacterium longum* transitional microorganism further encodes for G5_44.
8. The use according to any preceding clause, wherein the *Bifidobacterium longum* microorganism has an Average Nucleotide Identity (ANI) of at least 98% with at least one *Bifidobacterium longum* strain selected in the group consisting of CNCM I-5683, CNCM I-5684, CNCM I-5685, CNCM I-5686 and CNCM I-5687, and any combination thereof.
9. The use according to any preceding clause, wherein the glycan substrate is comprised in an ingredient.
10. The use according to clause 9, wherein the ingredient comprising the one or more glycan substrates is selected from the group consisting of purified polysaccharide or purified oligosaccharide, a dietary fibre ingredient, a semi-purified food ingredient, a raw food ingredient, a food additive, a semi-purified or purified peptido-glycan.
11. Use of a combination of a *Bifidobacterium longum* transitional microorganism and one or more glycan substrates to promote or assist the transition from a milk-based diet to solid food in an infant and/or in a young child, wherein the one or more glycan substrates is selected from the groups recited in any of Tables 1 to 3.
12. Use of a combination of a *Bifidobacterium longum* transitional microorganism and one or more glycan substrates to increase short-chain fatty acids (SCFA) production by the gut microbiome of an infant and/or in a young child, wherein the one or more glycan substrates is selected from the groups recited in any of Tables 1 to 3.
13. Use of a combination of a *Bifidobacterium longum* transitional microorganism and one or more glycan substrates to modulate the gut microbiome, wherein the one or more glycan substrates is selected from the groups recited in any of Tables 1 to 3.
14. The use according to any of clauses 11 to 13 wherein the *Bifidobacterium longum* transitional microorganism is capable of metabolizing said one or more glycan substrates.
15. Use of a glycan substrate to promote the growth of a *Bifidobacterium longum* transitional microorganism in the gut microbiota of an infant and/or of a young child, wherein the glycan substrate is selected from one or more of the glycan substrates selected from the groups recited in any of Tables 1 to 3.
16. A method of promoting the growth of a *Bifidobacterium longum* transitional microorganism in the gut microbiota of an infant and/or of a young child, the method comprising administering to the infant and/or to the young child a composition comprising one or more glycan substrates selected from the group recited in any of Tables 1 to 3.
17. A method of promoting/assisting the transition from a milk-based diet to solid food in an infant and/or in a young child, the method comprising administering to the infant and/or to the young child a combination of a *Bifidobacterium longum* transitional microorganism and one or more glycan substrates, wherein the one or more glycan substrates is selected from the groups recited in any of Tables 1 to 3 and the *Bifidobacterium longum* transitional microorganism is capable of metabolizing said one or more glycan substrates.
18. The use or method according to any of clauses 11 to 17, wherein the one or more glycan substrates is selected from the groups recited in Table 1 or 2 and the *Bifidobacterium longum* transitional microorganism is capable of metabolizing said one or more glycan substrates selected from the groups recited in Table 1 and Table 2
19. The use or method according to any of clauses 11 to 18, wherein the one or more glycan substrates selected from pectin, arabinogalactan and/or starch and the *Bifidobacterium longum* transitional microorganism is capable of metabolizing said glycan substrate.
20. The use or method according to any of clauses 11 to 19, wherein the *Bifidobacterium longum* transitional microorganism encodes one or more CAZymes selected from the group recited in Table 1, preferably wherein the *Bifidobacterium longum* transitional microorganism further encodes one or more CAZymes selected from the groups recited in Tables 2 and 3.
21. The use or method according to clause 20 wherein the *Bifidobacterium longum* transitional microorganism encodes (i) one or more CAZymes selected from the group recited in Table 1, and (ii) each of the CAZymes recited in Table 3 or each of the CAZymes recited in Table 3.
22. The use according to clause 21, wherein the *Bifidobacterium longum* transitional microorganism further encodes G5_44.
23. The use or method according to any of clauses 10 to 20, wherein the *Bifidobacterium longum* microorganism has an Average Nucleotide Identity (ANI) of at least 98% with at least one *Bifidobacterium longum* strain selected in the group consisting of CNCM I-5683, CNCM I-5684, CNCM I-5685, CNCM I-5686 and CNCM I-5687, and any combination thereof.
24. The use or method according to any of clauses 10 to 21, wherein the glycan substrate is comprised in an ingredient.
25. The use or method according to clause 24, wherein the ingredient comprising the one or more glycan substrates is selected from the group consisting of purified polysaccharide or purified oligosaccharide, a dietary fibre ingredient, a semi-purified food ingredient, a raw food ingredient, a food additive, a semi-purified or purified peptido-glycan.
26. A composition for promoting/assisting the transition from a milk-based diet to solid food in an infant and/or in a young child comprising a *Bifidobacterium longum* transitional microorganism and one or more glycan substrates selected from the group recited in any of Tables 1 to 3.
27. The composition according to clause 26, wherein the *Bifidobacterium longum* transitional microorganism is capable of metabolizing the one or more the glycan substrates selected from the groups recited in Tables 1 to 3
28. The composition according to clause 26 or 27, wherein the *Bifidobacterium longum* transitional microorganism is capable of metabolizing one or more glycan substrates contained in Table 1 or 2.
29. The composition according to any of clauses 27 or 28, wherein the *Bifidobacterium longum* the one or more glycan substrates is selected from pectin, arabinogalactan and/or starch.
30. The composition according to any of clauses 26 to 29, wherein the *Bifidobacterium longum* transitional microorganism encodes one or more CAZymes selected from Table 1, preferably wherein the *Bifidobacterium longum* transitional microorganism further encodes one or more CAZymes selected from Table 2 or 3.
31. The composition according to any of clauses 26 to 30, wherein the *Bifidobacterium longum* transitional microorganism encodes (i) one or more CAZymes selected from Table 1, and (ii) each of the CAZymes recited in Table 3 or each of the CAZymes recited in Table 3.
32. The use according to clause 31, wherein the *Bifidobacterium longum* transitional microorganism further encodes G5_44.
33. The composition according to any of clauses 26 to 31, wherein the *Bifidobacterium longum* microorganism has an Average Nucleotide Identity (ANI) of at least 98% with at least one *Bifidobacterium longum* strain selected in the group consisting of CNCM I-5683, CNCM I-5684, CNCM I-5685, CNCM I-5686 and CNCM I-5687, and any combination thereof.
34. The composition according to any of clauses 26 to 33, wherein the glycan substrate is comprised in an ingredient.
35. The composition according to any of clauses 26 to 34, wherein the ingredient comprising the one or more glycan substrates is selected from the group consisting of purified polysaccharide or purified oligosaccharide, a dietary fibre ingredient, a semi-purified food ingredient, a raw food ingredient, a food additive, a semi-purified or purified peptido-glycan.
36. Use of a composition according to any of clauses 26 to 35 to promote the growth of a *Bifidobacterium longum* microorganism that preferentially metabolizes one or more glycan substrates selected from the group recited in any of Tables 1 to 3.

Those skilled in the art will understand that they can freely combine all features of the present invention disclosed herein. In particular, features described for the product of the present invention may be combined with the method of the present invention and vice versa. Further, features described for different embodiments of the present invention may be combined. Where known equivalents exist to specific features, such equivalents are incorporated as if specifically referred to in this specification.

Further advantages and features of the present invention are apparent from the figures and non-limiting examples.

### Examples

### Example 1: Analysis of Carbohydrate Active Enzyme (CAZyme) genes of Bifidobacterium longum transitional microorganism

Genomes of *Bifidobacterium longum* subspecies listed in Figure 2 were annotated to CAZymes combining dbCAN2 (Zhang et al., Nucleic Acids Res. 46(W1):W95-W101 (2018)) tools and databases HMMdb (v9) and Diamond (v2.0.8). Query sequences with > 0.50 coverage and e-value < 1e-15 were annotated with HMMER according to the dbCAN CAZyme domain HMM database. Diamond was also used to annotate query sequences with hits in the CAZy database (Drula et al., Nucleic Acids Res. 50(D1):D571-D577 (2022)) (http://www.cazy.org/) with > 0.90 identity, and e value < 1e-102. HMMER annotation was prioritized and used in instances of mismatched CAZyme annotations of query sequences between HMMER and DIAMOND tools. Only CAZyme families and subfamilies encoding Glycoside Hydrolases (GHs) and Polysaccharide Lyases (PLs) were used for comparative analyses of *B. longum* subspecies (see Figure 2).

### Example 2: Utilization of glycan substrates

All bacterial strains retrieved from the Nestlé Culture Collection (NCC) were reactivated from a freeze-dried stock using two successive culturing steps (16 h, 37 °C, anaerobiosis) in MRS (De Man, Rogosa and Sharpe) media supplemented with 0.05 % cysteine (MRSc). Reactivated cultures were then centrifuged, washed, and resuspended in 1 volume of PBS.

For single culture *in vitro* experiments, washed bacterial cells from cultured isolates NCC5000, NCC5001, NCC5002, NCC5003, and NCC5004 were used to inoculate MRSc based medium without a carbon source (MRSc-C) (10 g/L of bacto proteose peptone n°3, 5 g/L bacto yeast extract, 1 g/L Tween 80, 2 g/L di-ammonium hydrogen citrate, 5 g/L sodium acetate, 0.1 g/L magnesium sulphate, 0.05 g/L manganese sulfate, 2 g/L di-sodium phosphate, 0.5 g/L cysteine) in which glucose, 2'FL, 3'FL, diFL, 3'SL, 6'SL, LNT, and LnNT were individually added as unique carbon source at a concentration of 0.5%. Growth was then performed in a 96 well microplate, with a volume of 200 µl per well. Incubation was performed in anaerobiosis (n=4 per testing group) for 48h, and optical density (OD) was measured in a spectrophotometer at 600 nm.

For *in vitro* tube fermentation experiments, pulverized or homogenized stool samples were mixed 10-fold by adding PBS/glycerol (1/10) (w/v) before centrifugation at 600 x g for 2 minutes. The slurry and pellet were then stored at -80°C. Frozen slurry of a fecal sample was thawed from storage and then use for the in vitro tube fermentation assays. The fecal slurry was inoculated with media based on that disclosed in Gibson et al. (Applied and Environmental Microbiology; 1988; 54(11); 2750-2755) with some modifications, specifically the exclusion of any carbon source. Specific glycans such as the HMOs 2'FL, 3FL, and fibers from larch wood, pea fiber, polydextrose, and corn bran fiber were added to the media as a single source of carbon and tested at 5 g/L each. *Bifidobacterium* isolates from the NCC (NCC5002 or NCC5004) were supplemented at 5E07 CFU/ml. A control group of the *in vitro* tube fermentation assays was performed under the same conditions with the corresponding added glycans where no Bifidobacterium isolates were supplemented (No suppl. group).

The *in vitro* tube fermentation assays (n=3 per testing group) were set up at 37°C with gentle stirring in an anaerobic environment (gas flow of pure N₂ and a mix of N₂, H₂, and CO₂ to ensure anaerobic conditions). Aliquots (0.5 mL) from fermentation tubes were taken and DNA was extracted, purified and analyzed at the time points indicated (0 hr, 24 hr and 48 hr). Quantification of *Bifidobacterium* isolates from the NCC (NCC5002 and NCC5004) was performed using isolate-specific primers in qPCR (triplicate qPCR reactions per sample), and absolute abundance was calculated based on amplicon number normalized to genome size. qPCR primer sets were designed and validated for specific quantification of each *Bifidobacterium* isolate (NCC5002 and NCC5004).

NCC5002 primers target a genomic region of 1484 bp within nucleotide positions 2112897 to 2111414:
Forward: GATTTTCTTGAGCAGCCTTTCG
Reverse: CTAGTTACCTGGGCGGAGCC

NCC5004 primers target a genomic region of 2284 bp within nucleotide positions 1153272 to 1155555:
Forward: AACGGTCTCAGCGGTGTATCC
Reverse: CGAAACAGCCAACGAGGTG

Aliquots (0.5 mL) from fermentation tubes were also taken for measurements of short-chain fatty acids (SCFA) using ¹H-nuclear magnetic resonance (¹H-NMR). Briefly, aliquots were centrifuged at 2,800 x g for 5 min and supernatant stored at -80 °C. For SCFA analysis, the frozen supernatant was left to thaw at room temperature and then centrifuged for 10 min at 10,000 x g at 4°C. 300 mL of the supernatant was mixed with 300 mL of 0.075 M sodium phosphate buffer (pH 7.4), and 560 mL was transferred to 5 mm ¹H-NMR tubes. The samples were then analyzed by ¹H-NMR experiments recorded at 300 K in a 600 MHz Bruker spectrometer. Acquisitions of ¹H-NMR spectra and pre-processing were performed using TopSpin 4.0.9 (Bruker BioSpin, Rheinstetten, Germany). Metabolite annotations were based on a comparison of recorded ¹H-NMR signals with metabolite signals from a BBIOREFCODE database (Bruker BioSpin, Rheinstetten, Germany) and public databased Human Metabolome Database (HMDB) (Wishart et al., Nucleic Acids Research, 50(D1), D622-D631(2021)). The measured ¹H-NMR spectra were imported into R statistical software environment (version 4.1.1) using the AlpsNMR package (Madrid-Gambin et al., Bioinformatics, 36(9), 2943-2945 (2020)). ¹H-NMR targeted signal integration was performed using a numeric integration automated routine in R statistical software. In particular, signals of butyrate at 0.90 ppm, propionate at 1.06 ppm, and acetate at 1.92 ppm were selected for integration.

Transitional *B. longum* isolates (NCC5002, NCC5001, NCC5003, NCC5000, NCC5004) have the genomic capacity to utilize neutral fucosylated HMOs, sialylated HMOs, and neutral non-fucosylated HMOs based on the presence of CAZymes that can target these types of glycans (Figure 3A). OD measurements at 48 hrs of culture with single HMOs show differential growth between transitional *B. longum* isolates (NCC5000, NCC5001, NCC5002, NCC5003, NCC5004) towards tested glycans, validating the prediction of growth from these glycans based on CAZyme profiles (Figure 3B). Supplementation of the transitional *B. longum* isolate NCC5004 to a complex fecal microbial community and tested in an *in vitro* tube fermentation assay against two neutral fucosylated HMOs, 2'FL and 3 FL, show significant growth after 24 hr and 48 hrs for both of these glycans compared to baseline (0 hrs) (Figure 3C). Together, these findings indicate that different HMOs (neutral fucosylated, sialylated and neutral non-fucosylated) promote the growth of transitional *B. longum* when grown in single culture and in a complex community.

To test the effects of different food glycans on the growth of transitional *B. longum,* two isolates were selected (NCC 5002 and NCC5004) with different capacities to digest dietary fibers based on their CAZyme profiles, with NCC5004 having a more extensive CAZyme repertoire than NCC5002 (Figure 4A). Supplementation of the transitional *B. longum* isolates NCC5002 (Figure 4B) or NCC5004 (Figure 4C) to a complex fecal microbial community and tested in an *in vitro* tube fermentation assay against four dietary fiber ingredients (larch wood fiber, pea fiber, polydextrose and corn bran fiber) show significant growth after 24 hrs and/or 48 hrs for most of these glycans with the exception of corn bran fiber in the case of NCC5002, and after 24 hrs and 48 hrs for all of these glycans in the case of NCC5004, compared to baseline (0 hrs). The dietary fiber ingredients tested were enriched in arabinogalactan, arabinan, alpha-glucans, and arabinoxylan, respectively.

To test the effects of combining transitional *B. longum* with specific glycans (dietary fibers or HMOs) on SCFA production we performed ¹H-NMR on the supernatant from *in vitro* tube fermentation assays at timepoints 0 hr (baseline), 24 hrs, and 48 hrs. ¹H-NMR measurements of SCFAs in the supernatant of *in vitro* fermentation assays showed that combination of glycans, either dietary fibers (Figures 5A and 5B) or HMOs (Figure 5C) with transitional *B. longum* increase the total abundance of SCFA produced by the fecal microbial community at 24 hrs and 48 hrs compared to baseline (0 hr).

Together, these findings validate the use of CAZyme profiles to identify fiber ingredients that have the potential to promote the growth of transitional *B. longum* when supplemented in a complex microbial community and increase the production of SCFAs.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. Use of a *Bifidobacterium longum* transitional microorganism to metabolize one or more glycan substrates, preferably wherein the glycan substrate is selected from the groups recited in any of Tables 1 to 3.

2. Use of a *Bifidobacterium longum* transitional microorganism according to claim 1, wherein the *Bifidobacterium longum* transitional microorganism is used to promote or assist the transition from a milk-based diet to solid food in an infant and/or in a young child.

3. Use of a combination of a *Bifidobacterium longum* transitional microorganism and one or more glycan substrates to promote or assist the transition from a milk-based diet to solid food in an infant and/or in a young child, wherein the one or more glycan substrates is selected from the groups recited in any of Tables 1 to 3.

4. Use of a combination of a *Bifidobacterium longum* transitional microorganism and one or more glycan substrates to increase short-chain fatty acids (SCFA) production by the gut microbiome of an infant and/or in a young child, wherein the one or more glycan substrates is selected from the groups recited in any of Tables 1 to 3.

5. Use of a combination of a *Bifidobacterium longum* transitional microorganism and one or more glycan substrates to modulate the gut microbiome, wherein the one or more glycan substrates is selected from the groups recited in any of Tables 1 to 3.

6. Use of a glycan substrate to promote the growth of a *Bifidobacterium longum* transitional microorganism in the gut microbiota of an infant and/or of a young child, wherein the glycan substrates is selected from one or more of the glycan substrates selected from the group recited in any of Tables 1 to 3.

7. A use according to any of claims 3 to 6 wherein the *Bifidobacterium longum* transitional microorganism is capable of metabolizing said one or more glycan substrates.

8. A method of promoting the growth of a *Bifidobacterium longum* transitional microorganism in the gut microbiota of an infant and/or of a young child, the method comprising administering to the infant and/or to the young child a composition comprising one or more glycan substrates selected from the group recited in any of Tables 1 to 3.

9. A method of promoting/assisting the transition from a milk-based diet to solid food in an infant and/or in a young child, the method comprising administering to the infant and/or to the young child a combination of a *Bifidobacterium longum* transitional microorganism and one or more glycan substrates, wherein the one or more glycan substrates is selected from the groups recited in any of Tables 1 to 3 and the *Bifidobacterium longum* transitional microorganism is capable of metabolizing said one or more glycan substrates.

10. A composition for promoting/assisting the transition from a milk-based diet to solid food in an infant and/or in a young child comprising a *Bifidobacterium longum* transitional microorganism and one or more glycan substrates wherein the one or more glycan substrates is selected from the groups recited in any of Tables 1 to 3.

11. Use of a composition according to claim 9 to promote the growth of a *Bifidobacterium longum* microorganism that preferentially metabolizes one or more glycan substrates selected from the group recited in any of Tables 1 to 3.

12. A use, method or composition according to any preceding claim, wherein the one or more glycan substrates is selected from the groups recited in Table 1 or 2 and the *Bifidobacterium longum* transitional microorganism is capable of metabolizing said one or more glycan substrates selected from the groups recited in Table 1 and Table 2.

13. A use, method or composition according to any preceding claim, wherein the *Bifidobacterium longum* transitional microorganism encodes one or more CAZymes selected from the group recited in Table 1, preferably wherein the *Bifidobacterium longum* transitional microorganism further encodes one or more CAZymes selected from Table 2 and 3.

14. A use, method or composition according to any preceding claim, wherein the *Bifidobacterium longum* microorganism has an Average Nucleotide Identity (ANI) of at least 98% with at least one *Bifidobacterium longum* strain selected in the group consisting of CNCM I-5683, CNCM I-5684, CNCM I-5685, CNCM I-5686 and CNCM I-5687, and any combination thereof.

15. A use, method or composition according to any preceding claim, wherein the glycan substrate is comprised in an ingredient, suitably wherein the ingredient is selected from the group consisting of purified polysaccharide or purified oligosaccharide, a dietary fibre ingredient, a semi-purified food ingredient, a raw food ingredient, a food additive, a HMO, a semi-purified or purified peptido-glycan.
